# EUROPEAN PATENT SPECIFICATION

(11) **EP 3 473 709 B1**
(45) Date of publication and mention of the grant of the patent: **08.12.2021**
(21) Application number: 18199472.4
(22) Date of filing: 09.10.2018
(51) Int. Cl.: C12N 9/18, C12N 15/85, C12N 15/90

(54) **DNA VECTOR CONTAINING A GENE ENCODING AN EQUINE BUTYRYLCHOLINESTERASE, FOR TRANSFORMATION AND INTEGRATION INTO LEISHMANIA TARENTOLAE CHROMOSOME.**
DNA-VEKTOR ENTHALTEND EIN GEN, DAS FÜR EINE EQUINE BUTYRYLCHOLINESTERASE KODIERT, ZUR TRANSFORMATION UND INTEGRATION IN DAS CHROMOSOM VON LEISHMANIA TARENTOLAE.
VECTEUR ADN CONTENANT UN GÈNE CODANT POUR UNE BUTYRYLCHOLINESTÉRASE ÉQUINE, POUR TRANSFORMATION ET INTÉGRATION DANS LE CHROMOSOME DE LEISHMANIA TARENTOLAE.

(30) Priority: 09.10.2017 PL 42311617
(43) Date of publication of application: 24.04.2019
(73) Proprietor: Uniwersytet Gdanski, 80-309 Gdansk (PL); Gdanski Uniwersytet Medyczny, 80-210 Gdansk (PL); Genozym sp. z o.o., 80-303 Gdansk (PL)
(72) Inventor: Skowron, Piotr, 80-177 Gdansk (PL); Jasiecki, Jacek, 80-344 Gdansk (PL)
(74) Representative: Pawlowska, Justyna

(56) References cited:
- EP-B1- 1 242 602
- Monika Wierdl ET AL: "Isolation and characterization of a cDNA encoding a horse liver butyrylcholinesterase: Evidence for CPT-11 drug activation", Biochemical Pharmacology, 1 January 2000 (2000-01-01), pages 773-781, XP055567825, England DOI: 10.1016/S0006-2952(99)00389-5 Retrieved from the Internet: URL:https://ac.els-cdn.com/S00062952990038 95/1-s2.0-S0006295299003895-main.pdf?_tid= 20daab38-9523-4291-8603-945a0f221237&acdna t=1552393569_e5bba8d9000f21fe407e7f2369bf1 839
- ERIK C RALPH ET AL: "His-tag truncated butyrylcholinesterase as a useful construct for in vitro characterization of wild-type and variant butyrylcholinesterases", PROTEIN EXPRESSION AND PURIFICATION, vol. 80, no. 1, 23 July 2011 (2011-07-23), pages 22-27, XP028299286, ISSN: 1046-5928, DOI: 10.1016/J.PEP.2011.07.005 [retrieved on 2011-07-23]
- GIANCARLO BASILE ET AL: "Recombinant Protein Expression in Leishmania tarentolae", MOLECULAR BIOTECHNOLOGY, vol. 43, no. 3, 1 November 2009 (2009-11-01), pages 273-278, XP055059760, ISSN: 1073-6085, DOI: 10.1007/s12033-009-9213-5
- STEPHAN KLATT ET AL: "Generation and Characterization of a Leishmania tarentolae Strain for Site-Directed in Vivo Biotinylation of Recombinant Proteins", JOURNAL OF PROTEOME RESEARCH, vol. 12, no. 12, 23 October 2013 (2013-10-23), pages 5512-5519, XP055567345, ISSN: 1535-3893, DOI: 10.1021/pr400406c
- ANVARSADAT KIANMEHR ET AL: "Cloning and expression of codon-optimized recombinant darbepoetin alfa in Leishmania tarentolae T7-TR", PROTEIN EXPRESSION AND PURIFICATION., vol. 118, 3 November 2015 (2015-11-03), pages 120-125, XP055567715, SAN DIEGO, CA. ISSN: 1046-5928, DOI: 10.1016/j.pep.2015.10.013

## Description

The subject of the invention is a DNA vector for obtaining butyrylcholinesterase for transformation and integration with *Leishmania tarentolae* chromosome. ChE enzymes: acetylcholinesterase (AChE) and butyrylcholinesterase - BChE (EC 3.1.1.8) that play a biological role in neural signalling (AChE) and natural detoxification of the body (BChE), are characterized by the ability to break down a broad spectrum of compounds with ester bonds and bonds and inactivation of organophosphorus compounds ― "OP". Therefore, they found a number of civil and military applications, including in: detoxification after exposure to organophosphorus pesticides, after bites of snakes, insects, drug overdose, treatment of neurodegenerative diseases - among others dementia, alcoholism, drug addiction, Alzheimer's disease, traumatic nervous system damage. Military and dual-use applications include actions to protect ecosystems and people focusing on early warning and removal of toxic compounds. One of the biggest threats is contemporary dissemination and post-war remnants, including OP compounds. They are one of the strongest known poisons acting on ChE enzymes by phosphorylation of serine in the active centre. OPs are used as pesticides, as well as Chemical Warfare Agents (CWAs) belonging to the paralytic-convulsive group (including soman, sarin, tabun, VX). In contrast to the threat of pesticides, which can be estimated both in area and time, increasing threats to the use of CWAs are unpredictable as to the scale and moment of occurrence. From the perspective of human health protection, the most important factor in poisoning is the time from human contact with these compounds to the administration of compounds that inhibit OPs. Fast and sensitive detection of CWAs and OP pesticides is a key parameter to prevent poisoning. The equine BChE enzyme is a proven key element in the detection of CWAs and pesticides from the OP group. It is a serine hydrolase present in the tissues of animals. This enzyme can contain larger substrates or inhibitors in its active centre than AChE, an enzyme responsible for the hydrolysis of acetylcholine in the central nervous system and neuromuscular connections. AChE, because it is a key enzyme for the transmission of nerve impulses, is a specific target for OP pesticides and CWAs. Inhibition of the AChE enzymatic activity leads to cholinergic poisoning symptoms such as miosis, salivation, vomiting, bradycardia and respiratory centre paralysis. Due to the specific structure of the BChE active centre and the ability to bind a wide spectrum of chemical compounds, it finds application as a sensitive component of biosensors and is also an effective *antidote.* In 2010, the American Food and Drug Administration (FDA) approved the use of recombinant human BChE as a therapeutic used in the poisoning and prevention of poisoning by CWAs, OP pesticides, toxins and for attempts to treat neurodegenerative diseases.

Eddleston et al. Lancet 371: 597-607 (2008) reveals that there are around 200,000 deaths in the world due to OP pesticides poisonings each year, therefore they are a serious public health problem, especially in the rural areas of developing countries.

Blong et al. Biochem. J. (1997) 327, 747-757 discloses that the tetramerization domain of human BChE, located at the C-terminal end of the polypeptide, can be removed by genetic engineering while maintaining enzymatic activity of the protein unless more than 50 amino acid residues are deleted.

Wierdl et al., Biochem Pharmacol. 59: 773-781 (2000) discloses that modifications of human BChE potentially affecting the enzymatic activity, based on the use of glycosidase to remove post-translational modifications of glycosylation from a protein already formed *in vivo,* showed that BChE does not require further glycosylation for enzymatic activity. The enzyme obtained as a result of transcription-translation *in vitro,* lacking glycosylation during biosynthesis, also showed enzymatic activity, although it exhibited altered biochemical properties.

Main et al., Biochem J. 143: 733-744 (1974) discloses a procedure for isolating to a state of electrophoretic homogeneity of a natural (non-recombinant) BChE, obtaining 15-18 mg of protein, from 20 L of equine plasma. The procedure was based on fractionation with ammonium sulphate, ion-exchange chromatography on DEAE-Sephadex and preparative electrophoresis under non-denaturing conditions. For use as *an antidote* for humans, it would be necessary to use plasma of several horses, which makes this approach impractical because of the lack of availability of animal material in such large quantities, the procedure is uneconomical and difficult to accept for epidemiological and ethical reasons.

Kronman et al. Gene 121: 295-304 (1992) disclose the cloning in mammalian cell lines, expression under the control of viral promoters of cytomegalovirus, Rous sarcoma virus, and SV40, and secretion outside the cell of recombinant human BChE. The protein obtained in the form of a mixture of dimers, tetramers and variants of post-translational modifications showed enzymatic activity. However, recombinant mammalian cell culture is expensive, long-lasting and susceptible to microbial infections, making it impractical for industrial applications.

In the description of the invention EP0388906 and US 5,595,903, a sequence of human AChE has been disclosed, as determined by cloning and sequencing of cDNA. The coding of the biologically active AChE protein has been verified by cloning the cDNA into the pGEM vector containing the SP₆ promoter, performing *in vitro* SP₆ RNA transcription by polymerase, micro injection into *Xenopus* oocytes, and conducting enzymatic tests for the presence of AChE activity. The methodology used allowed to demonstrate only on an analytical scale obtaining an actively biological cDNA clone of human AChE.

Wei et al. Biochem. Pharmacol. 60: 121-126 (2000) disclose the cloning of a gene encoding human BChE in insect larvae - *Bombyx mori* mulberry silkworm, infected with modified baculovirus viruses and obtaining a biologically active enzyme on an analytical scale. Insect cell culture has similar constraints as mammalian cell culture.

Mor et al. Biotechnol. Bioeng. 75: 259-266 (2001) disclose the construction of a transgenic plant comprising cloned and expressed in potatoes, a gene coding for the biologically active isoform of human AChE, exhibiting stability and retaining kinetic parameters corresponding to the human enzyme. The method has a number of advantages compared to recombinant tissue cultures or methods of separation of plasma, e.g. no epidemiological limitations and susceptibility to infection. The disadvantage is the slow growth rate of recombinant plants.

Lockridge et al. J. Med. Chem. Biol. Radiol. Def. 3: nihms5095 (2005) discloses a method for isolating natural human BChE on a preparative scale from 100 L of plasma. The method includes the steps of ion-exchange chromatography onto Q-Sepharose at low pH (4), affinity chromatography on procainamide-Sepharose, DEAE 8HR-HPLC, allowing a highly purified enzyme to be obtained with a low endotoxin content that meets the OP protection test criteria when tested on monkeys. A serious disadvantage of the method is: very limited availability of the amount of human plasma necessary to carry out the industrial process and epidemiological considerations.

Cerasoli et al. Chem. Biol. Interact. 157-158: 363-365 (2005) disclosed the cloning and expression of BChE in transgenic goats secreting BChE in milk. The recombinant BChE protein preparation was commercialized under the name Protexia (Nexia Biotechnologies, Que., Canada). The technical problem in the industrial process is the limited number of transgenic animals and the slow rate of their multiplication.

Saxena et al. J. Mol. Neurosci. 30: 145-148 (2006) discloses that BChE by binding toxic compounds present in plasma, hydrolyses them or binds permanently preventing them from reaching AChE present in peripheral nerve connections and the central nervous system. 1 mole of human BChE binds 1 mole of OP compounds, and a dose of 200 mg of this protein is able to prevent the effects of human intoxication weighing 70 kg with soman in the amount of 2× LD₅₀. Experiments on animal models that have been pre-treated with BChE showed full protection at a dose of 5x LD50 of the CWA administered. An important aspect is the amount of protein necessary to produce *the antidote* effect - BChE needed for one man to inactivate the toxin - 200 mg of pure BChE preparation is a very large amount, and its isolation by traditional methods (from equine plasma) is very expensive.

The description of the invention US 6,987,211has disclosed cloning and expression of a gene encoding a non-synaptic splicing variant of human AChE into transgenic mice under the control of the cytomegalovirus promoter. The genetic structure led to the expression and secretion of the human version of AChE in mouse milk. The same patent application discloses cloned and expressed using the above-mentioned methods: human AChE and BChE in a variant of the natural gene and by the expression of synthetic genes having deletions and point mutations as well as cloned and expressed CHE derived from insects. The presented method has been designed to be used for analytical research purposes, which does not meet the requirements of industrial scaling.

Evron et al. FASEB J. 21: 2961-2969 (2007) disclose the cloning of human AChE in *Nicotiana benthamiana,* a tobacco-related plant. The isolated recombinant enzyme was biochemically indistinguishable from the enzyme obtained from mammalian cell cultures. Used in even molar doses, it eliminated completely short-lived symptoms of OP poisoning in mice. The limitation of the method is the slow growth rate of plants.

Li et al. Chem. Biol. Interact. 187: 101-105 (2010) reveal a high level of expression of the cloned gene encoding human BChE in cells of insect larvae infected with modified baculovirus viruses. The method brings the above-mentioned limitations in the use of tissue cultures in industrial processes.

Morel and Massoulie Biochem. J. 328: 121-129 (1997) disclose the cloning and expression of AChE and its deletion derivatives of a rat and a snake in yeast *Pichia pastoris,* producing recombinant monomeric and dimeric forms, showing biological activity. The production level of recombinant AChEs was not more than 1 mg of protein from 1 L of culture. The obtained genetic structure leads to the expression of AChE at a very low level, since typically *Pichia pastoris* allows production of even more than 300 mg from 1 L of culture, thus the genetic constructs described are not suitable for industrial scaling.

Publication of M Wierdl et. al.: Isolation and characterization of a cDNA encoding a horse liver butyrylcholinesterase: evidence for CPT-11 drug activation, Biochem Pharmacol 2000 Apr 1;59(7):773-81 discloses that cDNA encoding a horse BuChE has been isolated. Based upon the NH2-terminal amino acid sequence of a purified horse BuChE, degenerate primers to amplify the coding sequence from horse liver cDNA was also designed. Following polymerase chain reaction and rapid amplification of the cDNA ends, an 1850-bp DNA fragment, containing an 1806-bp open reading frame was generated.

Erik C Ralph et al in publication His-tag truncated butyrylcholinesterase as a useful construct for in vitro characterization of wild-type and variant butyrylcholinesterases; Protein Expr Purif 2011 Nov;80(1):22-7 discloses expression and purification of recombinant BChE proteins and its expression, in a viral expression system, wile BChE proteins having C-terminal truncation of the tetramization domain and a His-tag.

Using host cell systems for expression of recombinant proteins are known. EP1242602 discloses a method of recombinant protein production in vitro with cultivated non pathogenic Kinetoplastidae parasites included *Leishmania tarentolae.* This includes description of cultivation conditions for several species that allow large scale growth of these organisms on cheap media. Codon optimization using codons for *L. torentolae* is also-known from eg. Stephan Klatt el Generation and Characterization of a Leishmania tarentolae Strain for Site-Directed in Vivo Biotinylation of Recombinant Proteins, J. Proteome Res. 2013, 12, 12, 5512-5519. Vectors for transformation and integration with better system for culturing like *Leishmania tarentolae* require modification for the particular system.

So far, the attempts of efficient expression of genes encoding ChE, and biosynthesis of biologically active ChE enzymes in microorganisms commonly used for industrial, efficient protein production, such as *Escherichia coli* or yeast bacteria, have failed or only partially succeeded. This is due to a number of difficulties: rapid ChE proteolysis in bacteria and other microorganisms, the presence of many disulphide bridges in CHE, impossible to properly form in prokaryotic microorganisms, and necessary to achieve active conformation by these enzymes, difficulties in correct protein folding to enzymatic-active conformation, and lack of required mechanisms for CHE post-translational modifications. Expression strategies in systems functionally corresponding to microbial cultures - cell suspensions of eukaryotic organisms: insects, baculovirus infected and mammalian cell lines are difficult for industrial applications due to high costs, the need to adapt difficult stages to automation, long incubation period, susceptibility to bacterial infections.

The object of the invention is to provide DNA vectors for transformation and integration with better system for culturing like *Leishmania tarentolae* by designation of the vector with particular changes in the structure to be expressed strictly in *Leishmania torentolae* allowing to obtain recombinant, biologically active equine BChE protein easily.

The subject-matter of the invention is a DNA vector for transformation and integration with *Leishmania tarentolae* chromosome containing a gene according to Seq.3 or Seq.7 or Seq.8 encoding a truncated version of equine BChE, devoid of the tetrameric domain, the vector being for biosynthesis of a recombinant protein from the Open Reading Frame contained in the gene, wherein gene contains the Open Reading Frame encoding a protein with Seq.1 containing 564 amino acid residues and bearing at an N-terminus a secreted *Leishmania sequence* with 23 amino acid residues and additionally containing 2 Gly-Ala amino acid residues, and at the C-terminus containing 4 Leu-Lys-Gly-Thr amino acid residues and a 6-residue His-tag.

Preferably, the DNA vector is a shuttle vector for cloning and expression of protein, further comprising a minimum of two origin of replication, preferably for *Escherichia coli* and / or *Leishmania tarentolae.*

Preferably, the DNA vector comprises a secretory signal coding sequence, preferably *Leishmania* in origin, antibiotic resistance gene, preferably encoding bleomycin and / or ampicillin, transcription promoter, preferably T7 bacteriophage, transcription repressor gene, preferably regulated by tetracycline, operator gene, preferably regulated by a tetracycline repressor, a translation initiation signal, a coding sequence for a translation stop signal, preferably a sequence encoding 6 histidine residues.

Expressive DNA vector for transformation and integration with *Leishmania tarentolae* chromosome, for the production of a protein with Seq.4, according to invention contains a total of 614 amino acid residues, including the additional 2 amino acid residues Met-Ala at the N-terminus, and with an additional 4 Leu-Lys-Gly-Thr amino acid residues and the 6-residue His-tag at the C-terminus, wherein the vector comprises a gene according to Seq.6 encoding equine BChE holoenzyme

DNA vector for transformation and integration with *Leishmania tarentolae* chromosome, for the production of protein with Seq.4, according to the invention contains a total of 614 amino acid residues, including the additional 2 amino acid residues Met-Ala at the N-terminus and bearing additional 4 amino acid residues Leu -Lys-Gly-Thr and 6-residue His-tag at the C-terminus, wherein the vector comprises a synthetic gene with a sequence of Seq.3 encoding the equine BChE holoenzyme.

Sequences' description is explained below.

Native gene encoding BChE butyrylcholinesterase shown in Seq.6 is known and desribed hereinafter as an example for comparison with the invention. The subject matter of the invention set the described constructs - DNA vectors - that comprises some known parts - sequences or it parts - but the each mentioned sequences and modification in one construct is new and set subject-matter of invention. The subject-matter is mostly described as a DNA vector for transformation and integration with *Leishmania tarentolae* chromosome that containing a gene according to Seq.3 or Seq. 6 or Seq.7 or Seq.8 encoding a truncated version of equine BChE with modification described according to other sequences decried heretofore - recombinant genes for production of recombinant BChE.
Seq.1 - amino acid sequence, forming the SP-BChE-His protein. SP - is the *Leishmania* secretion sequence, BChE - fragment of the synthetic gene, His - is the 6-residue His-tag
Seq.2 - amino acid sequence encoding the GA-BChE-His protein. GA - means 2 Gly-Ala residues, resulting from the construction of the pLEXSY-blecherry 3 vector and the beginning of the synthetic gene fragment sequence after cleavage of SP, His - is the 6-residue His-tag
Seq.3 - nucleotide sequence of the Open Reading Frame of a BChE fragment, devoid of a DNA fragment encoding the N-terminal equine secretory sequence and a C-terminal 45-amino acid tetramerization domain with a length of 1587 base pairs in a single-stranded form.
Seq.4 - the amino acid sequence that makes SP-BChE-HorseHis and SP-BChE-syntHis proteins. SP - means equine secretory sequence, BChE-Horse - full-length natural gene - known, BChE-synt - full-length synthetic gene, His - is the 6-residue His-tag
Seq.5 - the amino acid sequence that forms the mature BChE-HorseHis and BChE-syntHis proteins, indicated in Seq. 4 after cleavage of the equine signal sequence.
Seq.6 - nucleotide sequence of a PCR product, in single-stranded form encoding the SP-BChE-HorseHis protein (without sequence elements derived from the pLEXSY I-blecherry3 vector).
Seq.7 - nucleotide sequence of a PCR product, in a single-stranded form encoding the SP-BChE-syntHis protein (without sequence elements derived from the pLEXSY I-blecherry3 vector),
Seq.8 - the resulting PCR product with a length of a 1614 base pairs an Open Reading Frame of a synthetic BChE fragment, devoid of a DNA fragment encoding the N-terminal horse secretory sequence and a C-terminal 45 amino acid tetramerization domain, shown in single-stranded form.

Plasmid sequences as conformation of production of BChE:
Seq.9 - nucleotide sequence of plasmid pLEX-BCHE-ST in single-stranded form, with a length of 8807 base pairs. pLEX-BCHE-ST stands for the pLEXSY I-blecherry3 vector containing a cloned-in shortened synthetic BChE gene, bearing the *Leishmania* secretory sequence.
Seq.10 - nucleotide sequence of plasmid pLEXSY-BChEHor with a length of 8957 base pairs, in single-stranded form. pLEX-BChEHor stands for the pLEXSY I-blecherry3 vector containing the full-length natural equine BChE gene, bearing equine secretory sequence.
Seq.11 - nucleotide sequence of the plasmid pLEXSY-BChEEco, with a length of 8957 base pairs, in a single-stranded form. pLEX-BChEHor stands for the pLEXSY I-blecherry3 vector containing a fully cloned synthetic BChE gene, bearing equine secretory sequence.

The invention and its effect is illustrated by the following examples i and the figures described in the examples.

Example 1. of molecular cloning, expression and production of a shortened version of recombinant equine BChE and holoenzyme of full-length equine BChE in the microorganism *Leishmania tarantolae.*Just as information from the state of the art an example of cloning of a natural gene obtained from horse's cDNA is also presented. The present invention is versatile for use in molecular cloning, expression and production of recombinant proteinin the microorganism *Leishmania tarantolae.*

Protozoan *Leishmania tarentolae*, originally isolated from the intestins of the gecko *Tarentola mauritanica* is non-pathogenic for humans (Biosafety level 1), allowing for safe breeding in laboratory conditions. Based on the genetically modified *Leishmania tarentolae* protozoan, a commercial kit was developed for cloning and protein expression - LEXSY, by company: Jena Bioscience GmbH (Germany) and that is what has been used in the present invention. Important, from the point of view of the use for cloning and expression of eukaryotic proteins, is the property of having complicated mechanisms of folding and post-translational modification of proteins, similar to those existing in mammalian cells. The level of glycosylation and the branching process of multi-sugar chains differs from the bacterial (essentially - lack), the yeast (frequent hyperglycosylation) or the baculovirus systems (often incomplete glycosylation), but is similar to the mammalian type. The presence of glycosylation and its appropriate method are necessary for the proper folding of proteins and their enzymatic activities. Although *Leishmania tarentolae* is characterized by a much longer period of the experimental cycle than cloning and protein expression experiments in bacteria and yeast, due to much longer than the bacteria duration of one generation - about 4 hours, this disadvantage is compensated by a high level of protein expression, according to literature data exceeding 300 g / L culture, the ease of its secretion into the medium solution, and thus a perfectly convenient procedure for isolating recombinant protein and its proper folding into the biologically active form and high solubility. The invention uses an inducible version of the LEXSinduce3 system, the *Leishmania tarentolae* strain (LEXSY host T7-TR) which is characterized by an inducible system of overproduction of the cloned protein based on the T7 bacteriophage hybrid promoter and the operator coupled to it, derived from the regulatory region of the gene encoding the repressor, which controls tetracycline resistance. The level of biosynthesis of recombinant protein cloned in the above-mentioned system is strictly controlled by selecting the appropriate concentration of tetracycline. Such a precise possibility of matching the level of protein overproduction is particularly important when expressing proteins toxic to the recombinant host. *Leishmania tarentolae* T7-TR has a chromosome modification consisting in the integration of T7 RNA polymerase encoding genes, specific for the T7 promoter and the gene encoding the tetracycline repressor of transcription in the region of the *ssu* gene encoding the smaller subunit of ribosomal RNA. In this chromosomal location constitutive expression of both genes takes place, mediated by RNA polymerase of I *Leishmania tarentolae.* The stability of genetic modification is maintained by incorporating a hygromycin and nourseotricin antibiotic resistance cartridge in the similar chromosomal location of *Leishmania tarentolae* T7-TR. In addition to the specialized host, the pLEXSY-blecherry 3 shuttle vector, which is part of the above-mentioned set, capable of multiplying in *Escherichia coli* bacteria, was used for cloning and expression, thus offering the possibility of convenient preparation of the genetic construct and capable of chromosomal integration into *Leishmania tarentolae* T7-TR. According to the invention a DNA fragment, indicated in Seq.3 or Seq.6 or Seq.7 or Seq.8, was cloned into this vector, encoding BChE in such a way that it was in the correct orientation and Reading frame with respect to the T7 promoter and translation start signals. After the cloned BChE coding gene, in the correct orientation and bearing translation start signals, there was a gene derived from the vector used, encoding a blecherry reporter protein, the expression of which can be accurately monitored spectrophotometrically. The level of biosynthesis of the blecherry protein in this system correlates with the biosynthesis level of the cloned and expressed BChE protein gene, indicated in Seq.1 or Seq.2 or Seq.4 or Seq.5 in *Leishmania tarentolae* T7-TR. The expressive genetic construct was made in *Escherichia coli.* In the next step, the vector was linearized with a double cleavage of the Swal restriction enzyme and then introduced into *Leishmania tarentolae* T7-TR cells, where it was subjected to chromosomal integration in the region close to the *ssu* gene. Chromosomal integration is stable, additionally selectable by the gene coding for antibiotic-bleomycin resistance, located in an integrated vector.

Example 2. Design of a synthetic gene encoding a short version of equine BChE devoid of the tetramerization domain.

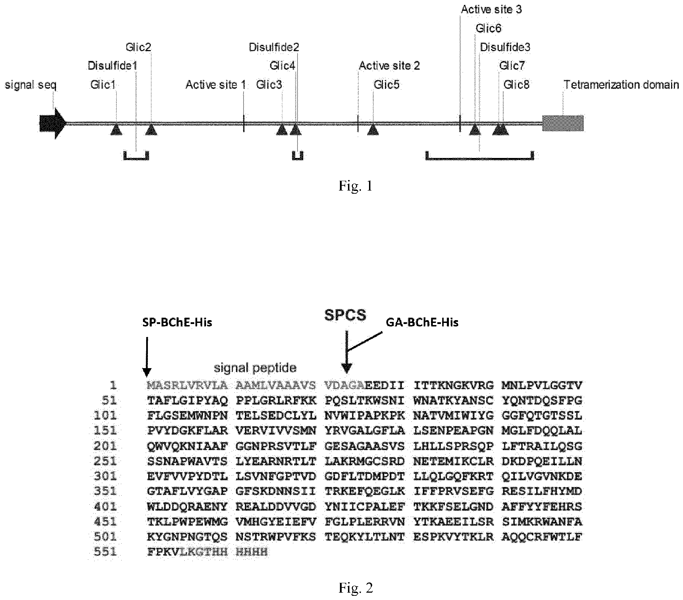

Based on the cDNA sequences available in public databases (*Equus caballus* BChE, cDNA, NCBI Reference Sequence: NM_001081850.1), a completely synthetic gene encoding the full-length BChE holoenzyme was designed. The nucleotide sequence at the DNA level was significantly different from the natural nucleotide sequence of the BChE horse gene, because it contained no introns, it included optimization of tertiary mRNA structures, the codon composition was also different, however, what was encoded was a polypeptide with an identical amino acid sequence like the natural enzyme - equine BChE whose expression method in the enzymatically active form is the main aspect of the invention. The universal scheme presented above has been used for molecular cloning and genetic expression of BChE gene variants: version of full-length BChE recombinant protein and a truncated variant in which parts of the sequence, not relevant for enzymatic activity, have been removed. Preferably, it is a version in which the biosynthesis, secretion and solubility of the recombinant protein were improved. For this purpose, the N-terminal amino acid signal sequence for extracellular secretion was removed, which is less efficient in directing extracellular secretion in *Leishmania tarentolae* and the C-terminal tetrameric domain relevant for *in vivo* functions in horse, but negligible for enzymatic protein activity for applications in biotechnology. The embodiment shown in Fig. 1 shows a map of a full-length synthetic gene of 1806 base pairs encoding recombinant equine BChE with an amino acid sequence of 602 amino acid residues that make up the 68.83 kDa protein corresponding to the sequence encoded by the horse cDNA, with marked functional elements: equine signal sequence (*signal peptide*), glycosylation points (Glic1-8), the area of disulphide bridges (*Disuffidel-3*), amino acid residues of the active centre (*Active site* 1-3), tetramerization domain (*Tetramerization domain*). In the embodiment shown in Fig. 2, the amino acid sequence of the recombinant bioactive BChE gene fragment is shown, wherein the equine secretory signal sequence has been replaced with the amino acid sequence of secretion signal (SP) of *Leishmania* (23 amino acid residues, encoded by 69 base pairs), additional 2 Gly-Ala amino acid residues, encoded by 6 base pairs, the addition of which results from the construction of the pLEXSY-blecherry 3 vector, amino acid sequence encoded by Open Frame Reading of the BChE fragment, devoid of the C-terminal 45 amino acid tetramerization domain (529 amino acid residues, encoded by 1587 base pairs), additional 4 Leu-Lys-Gly-Thr amino acid residues, encoded by 12 base pairs, the addition of which results from construction of the pLEXSY-blecherry 3 vector, 6-residue His-tag to isolate the recombinant protein by metal affinity chromatography, encoded by 18 pair of bases. In summary, the constructed fusion Open Reading Frame encodes 564 amino acid residues that form the SP-BChE-His fusion protein with a 63.66 kDa molecular mass, as indicated in Seq.1. During the process of maturation and secretion of protein to medium, the signal amino acid sequence for extracellular secretion is cut off, as a result of which a truncated GA-BChE-His fusion protein of 541 amino acid residues and 61.39 kDa molecular mass is formed, indicated in Seq.2. The nucleotide sequence of the Open Reading Frame of the BChE fragment, devoid of the DNA section encoding the N-terminal horse secretory sequence and the C-terminal 45-amino acid tetramerization domain of 1587 base pairs, in single-stranded form, is shown in Seq.3.

Example 3. Design of a natural and according to the invention synthetic gene encoding the BChE holoenzyme containing the tetramerization domain.

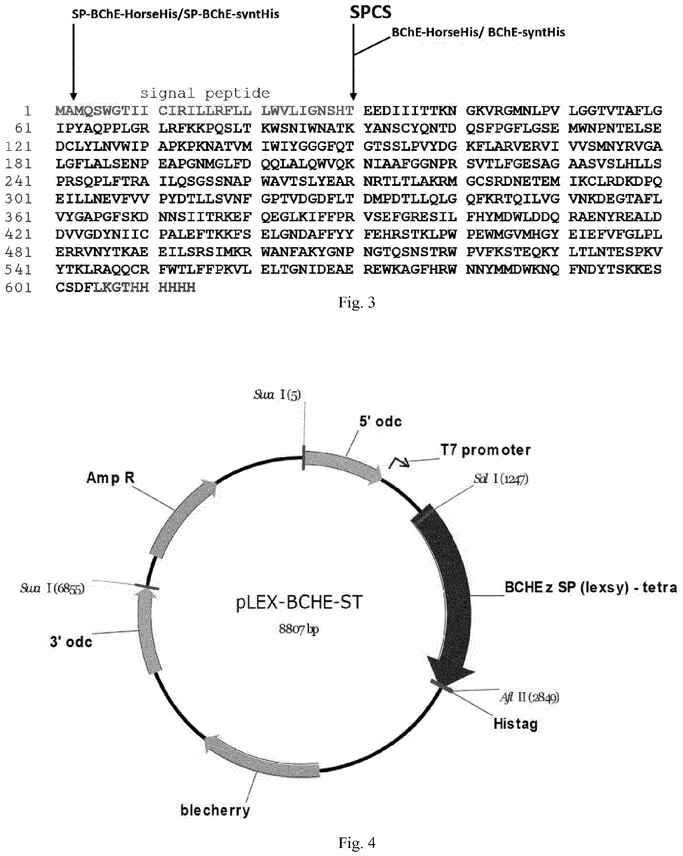

In the embodiment shown in Fig. 3, the amino acid sequence of the recombinant bioactive holoenzyme BChE protein is shown, where the equine secretion signal sequence (28 amino acid residues, encoded by 84 base pairs) is retained, preceded by additional 2 amino acid residues Met-Ala, encoded by 6 base pairs, the addition of which results from the construction of the pLEXSY-blecherry 3 vector, nucleotide sequence forming the Open Reading Frame, coding mature BChE holoenzyme (574 amino acid residues, encoded by 1722 base pairs), additional 4 Leu-Lys-Gly-Thr amino acid residues, encoded by 12 base pairs, the addition of which results from the construction of the pLEXSY-blecherry 3 vector, the 6-residue His-tag to isolate the recombinant protein by metal affinity chromatography, encoded by 18 base pairs. Concisely designed fusion Open Reading Frames consist of 1842 base pairs encoding 614 amino acid residues that form SP-BChE-HorseHis fusion proteins (obtained on the basis of cDNA) and SP-BChE-synt-His (on the basis of a synthetic gene) with molecular weight 70.66 kDa, indicated in Seq.1. During the process of protein maturation and secretion to medium, the signal amino acid sequence is cut off, as a result of which shortened BChE-HorseHis and BChE-synt-His fusion proteins with lengths of 584 amino acid residues and molecular weight of 67.15 kDa are formed. The amino acid sequence forming the SP-BChE-HorseHis and SP-BChE-syntHis proteins is shown in Seq.4; the amino acid sequence forming the mature BChE-HorseHis and BChE-syntHis proteins is shown in Seq.5; he nucleotide sequence of the PCR product, in the single-stranded form encoding the SP-BChE-HorseHis protein (without sequence elements derived from the pLEXSY I-blecherry3 vector), is shown in Seq.6; the nucleotide sequence of the PCR product, in the single-stranded form encoding the SP-BChE-syntHis protein (without sequence elements derived from the pLEXSY I-blecherry3 vector), is shown in Seq.7.

Example 4. Molecular construction and cloning of a shortened version of recombinant equine BChE protein.

Based on the nucleotide sequence of the synthetic gene encoding the mature (intron-free) version of the gene encoding the horse BChE indicated in the exemplary embodiments in Examples 1 and 2, a DNA replication reaction was performed by PCR method. A shortened fragment of the gene encoding BChE, shown in Fig. 1 and Fig. 2, was amplified using primers:
5'―ATAGTCGACGCTGGCGCCGAAGAAGACATCATCATCACCACC-3' and
5'-ATTCTTAAGAACTTTCGGGAAGAACAGGGTCCAGAAACGG-3'.

At the 5 'ends of the primers, nucleotide sequences (underlined), cleaved by SalI and MspCI restriction endonucleases, were inserted. The resulting PCR product, 1614 base pairs in length (shown in single-stranded form as Seq.8) contains the truncated BChE gene, which after cloning into the pLEXSY I-blecherry3 vector, encodes a fusion gene, ending with a 5 'nucleotide sequence encoding the *Leishmania* secretion signal - Met-Ala-Ser-Arg-Leu-Val-Arg-Val-Leu-Ala-Ala-Ala-Met-Leu-Val-Ala-Ala-Ala-Val-Ser-Val-Asp-Al a and two additional amino acid residues Gly-Ala, and at the 3' end it bears sequences encoding the 4 additional Lue-Lys-Gly-Thr amino acid residues and the 6-residue His-tag in place of the nucleotide sequence encoding the BChE tetramerization domain. Concisely designed Open Reading Frame encodes 564 amino acid residues, indicated in Seq.1. The obtained PCR amplification product was isolated using a set of mini silica columns for DNA purification after enzymatic reactions (Roche, Switzerland) and subjected to digestion with restriction endonucleases SalI and MspCI. The vector pLEXSY I-blecherry3 was also subjected to digestion with SalI and MspCI. The obtained digestion products were subjected to electrophoretic separation in a 1% agarose gel, from which the linear fragment of the pLEXSY I-blecherry3 vector was excised, containing the expression cassette and the digested PCR product. The DNA fragments were purified using a DNA isolation kit from an agarose gel (Roche, Switzerland). After purification, the linear vector pLEXSY I-blecherry3 and the digested PCR product were ligated using T4 bacteriophage DNA ligase (New England Biolabs, USA), maintaining continuity of the Open Reading Frame with the 6-residue Histidine-tag incorporated at the C-terminus of the polypeptide. The resulting plasmid pLEX-BCHE-ST, with a length of 8807 base pairs, is illustrated in Fig. 4, indicating: the T7 bacteriophage promoter controlling BChE biosynthesis (broken arrow / T7 promoter), the nucleotide sequences recognized by the selected restriction endonucleases (SalI, AflII, Swal), shortened gene encoding BChE fragment (BChE with SP (lexsy) -tetra), 6-residue His-tag (His tag), a blecherry reporter gene (blecherry), nucleotide sequences of the region of the *ssu* gene, encoding a smaller subunit of ribosomal RNA (5 'seq and 3 ' seq), ampicillin resistance gene (Amp R). The vector obtained from the ligation was subjected to restriction analysis, and the cloned fragment of the BChE gene was sequenced to confirm obtaining the designed vector construct. The nucleotide sequence of the plasmid pLEX-BCHE-ST in a single-stranded form, with a length of 8807 base pairs, is shown in Seq.9.

Example 5. Construction and molecular cloning of a holoenzyme of a natural for additional examples and synthetic gene encoding equine BChE holoenzyme containing a tetrameric domain according to the invention.

Based on the nucleotide sequence of the synthetic gene and the nucleotide sequence of the natural cDNA, encoding the mature (intron-free) versions of the gene encoding equine BChE, indicated in the exemplary embodiments in Examples 1, 2 and 3, the DNA replication reaction was performed using the PCR technique of full-length genes encoding BChE - natural and synthetic one, shown in Figures 1, 3 and 5. The synthetic gene was amplified using primers:
5'― TAGCCATGGCCATGCAGTCCTGGGGTACCATC -3' and
5'-TATTCTTAAGGAAGTCGGAGCAGGATTC-3'.

The gene obtained on the basis of cDNA was amplified using primers:
5'― TAGCCATGGCCATGCAGAGCTGGGGTACAATC -3' and
5'- ACTCTTAAGAAAATCTGAACAGCTTTCTTTCTTGCTAGTG-3'.

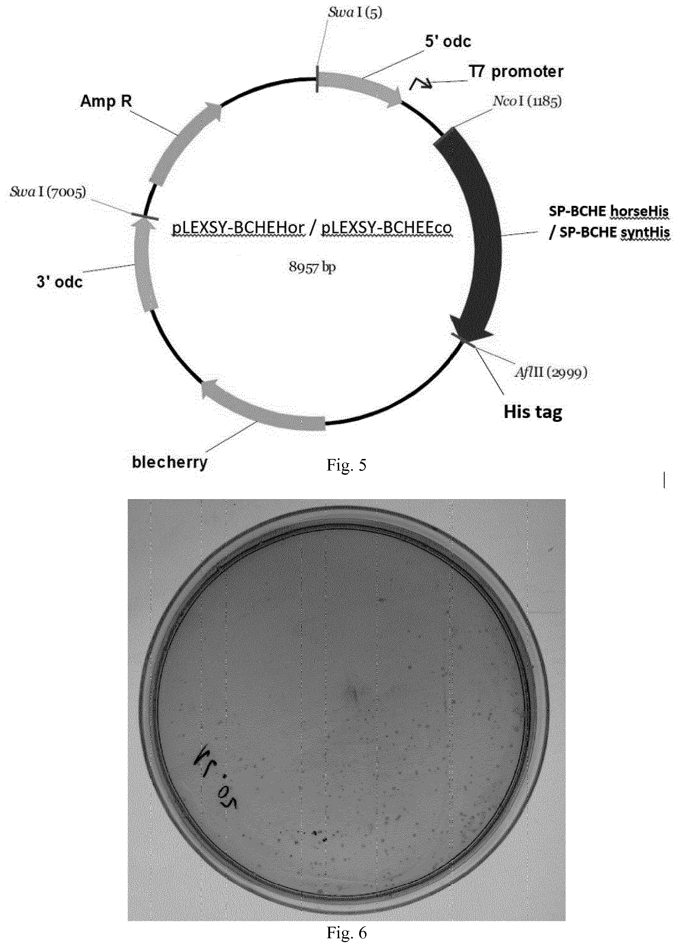

At the 5' ends of the primers, nucleotide sequences (underlined), cleaved by Ncol and MspCI restriction endonucleases, were inserted. The resulting PCR products with a length of 1826 base pairs (on the cDNA template) and 1827 base pairs (on the template of the synthetic BChE gene) contain a complete Open Reading Frame of the full-length BChE gene, which after cloning into the pLEXSY I-blecherry3 vector, encodes a fusion gene bearing the the 5 'end of the nucleotide sequence encoding the *Leishmania* secretion signal - Met-Ala-Ser-Arg-Leu-Val-Arg-Val-Leu-Ala-Ala-Ala-Met-Leu-Val-Ala-Ala-Ala-Val-Ser-Val-Asp-Ala and two additional Gly-Ala amino acid residues, and at the 3' end, bearing sequences encoding 4 additional Leu-Lys-Gly-Thr amino acid residues and a 6- residue His-tag. The obtained PCR amplification products were isolated using a set of mini silica columns for DNA purification after enzymatic reactions (Roche, Switzerland) and subjected to digestion with restriction endonucleases SalI and MspCI. The vector pLEXSY I-blecherry3 was also subjected to digestion with SalI and MspCI. The obtained digestion products were subjected to electrophoretic separation in a 1% agarose gel, from which the linear fragment of the pLEXSY I-blecherry3 vector was excised, containing the expression cassette and the digested PCR product. The DNA fragments were purified using a DNA isolation kit from an agarose gel (Roche, Switzerland). After purification, the linear vector pLEXSY I-blecherry3 and the digested PCR product were subjected to ligation using T4 bacteriophage DNA ligase (New England Biolabs USA). The resulting construct retains the continuity of the BChE Open ReadingFrame with the 6-residue His-tag incorporated at the C-terminus of the polypeptide. The resulting plasmids pLEX-BChEHor (obtained on the basis of cDNA) and pLEX-BChEEco (on the basis of a synthetic gene) containing two variants of full-length genes encoding BChE holoenzymes are illustrated in Fig. 5, indicating: T7 bacteriophage promoter controlling BChE holoenzymes (SP-BCHE horseHis, or SP-BCHE-syntHis, indicated in Seq.4), 6-residue His-tag (His tag), a blecherry reporter gene (blecherry), nucleotide sequences of the region of the *ssu* gene, encoding a smaller subunit of ribosomal RNA (5 'seq and 3 ' seq), ampicillin resistance gene (Amp R). The vector obtained from the ligation was subjected to restriction analysis, and the cloned BChE genes were sequenced to confirm obtaining the designed vector constructs. The nucleotide sequence of the pLEXSY-BChEHor plasmid, with a length of 8957 base pairs, in a single-stranded form, is shown in Seq.10; the nucleotide sequence of the pUCXEE-BChEEco plasmid with a length of 8,957 base pairs, in a single-stranded form, is shown in Seq.11.

Example 6. Transformation of *Leishmania tarentolae* T7-TR with the constructed vectors carrying expression constructs encoding holoenzymes and a shortened BChE gene variant.

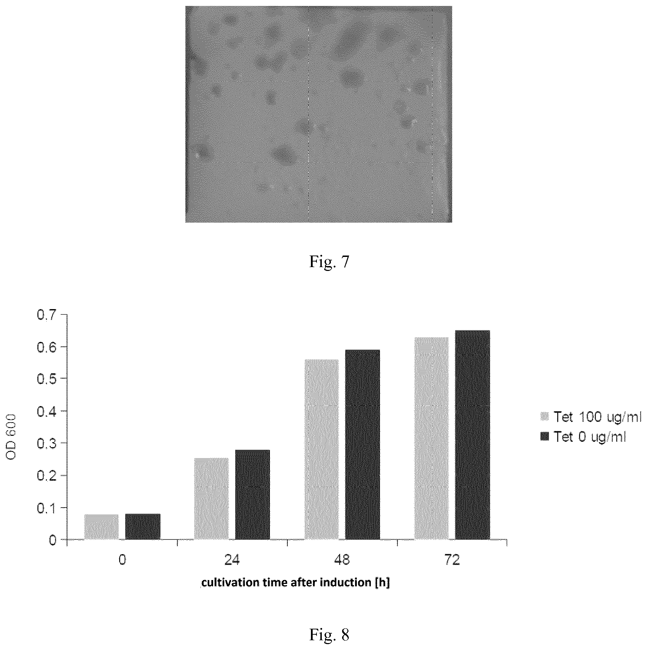

In the exemplary embodiment shown in Fig. 4 and Fig. 5, constructed vectors carrying the truncated BChE variant and the BChE holoenzymes indicated in the exemplary embodiment in Examples 1,2,3,4,5 were subjected to transformation by the electroporation method. For this purpose, the indicated DNA vectors were cut with SwaI restriction endonuclease to obtain linear forms. The digestion products were subjected to electrophoresis in a 1% agarose gel, and then fragments containing the BChE genes' expression modules and the nucleotide sequences of the *ssu* gene region were isolated from the gel. These fragments were introduced into *Leishmania tarentolae* T7-TR cells by an electrical impulse of 450 V, lasting 5.4 ms, with the capacitance of 450 µF of a capacitor used. The transformed *Leishmania tarentolae* T7-TR cells were incubated for 20 hours at 26°C in BHI medium and then plated on Petri dishes containing BHI medium solidified with agar, supplemented with selective antibiotic - bleomycin. Incubation was carried out for 8 days at 26°C. In the exemplary embodiment presented in Fig. 6, after 8 days, *Leishmania tarentolae* T7-TR colonies transformed with the vectors indicated in Examples 1,2,3,4 were observed on the plate. In order to verify the expression of the cloned BChE variants, the level of the coupled expression of the blecherry reporter gene was monitored. An imprint of the culture from a Petri dish on a nitrocellulose membrane was made, containing colonies of transformed *Leishmania tarentolae* T7-TR and the membrane was placed in a Petri dish containing BHI medium solidified with agar, supplemented with bleomycin and induction of transcription of hybrid T7 promoter - tetracycline at a concentration of 100 µg / ml. In the embodiment shown in Fig. 7, after 4 days, *Leishmania tarentolae* T7-TR colonies transformed with the vectors indicated in Examples 4 and 5 were observed on the plate, which turned pink to indicate the expression of the blecherry reporter gene coupled to the BChE genes. Due to the possibility of integration into the *Leishmania tarentolae* T7-TR chromosome of various copies of the expression module of the vectors indicated in Examples 4 and 5, and thus the expected different levels of blecherry and BChE expression, selected for further analysis were the colonies showing the darkest pink shade correlated with the level of transcription induction from the T7 hybrid promoter and blecherry biosynthesis.

Example 7. Overproduction of recombinant BChE in *Leishmania tarentolae* cells.

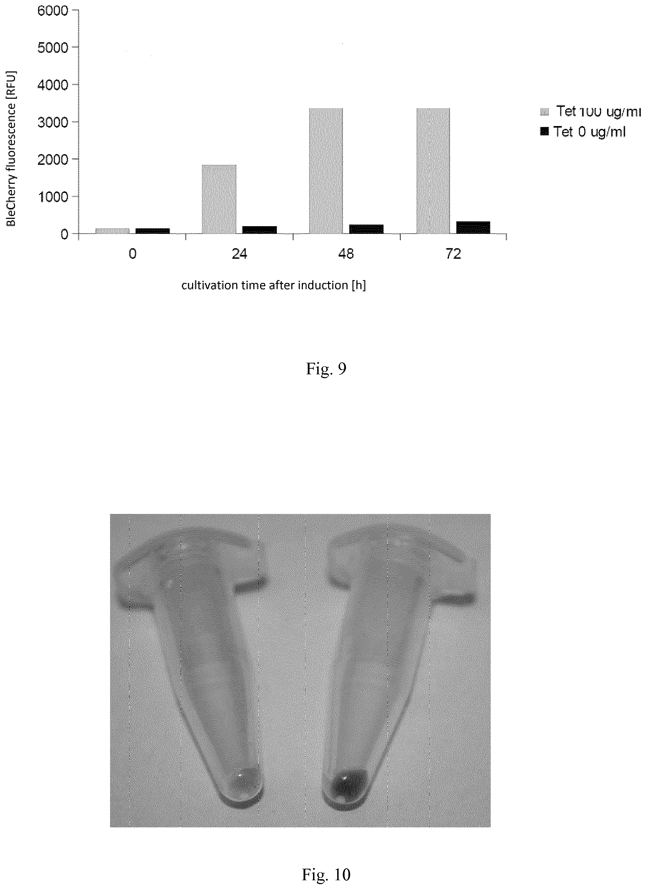

In the exemplary embodiment presented in Example 6, several dozens of clones differing in the expression level of the blecherry reporter gene have been obtained as a result of the integration of a different number of copies of the expression cassette containing the gene encoding the shortened version of BChE into the genome of *Leishmania tarentolae* T7-TR clones. The selected clone, with strong - visually assessed - blecherry expression, was used to inoculate 10 ml of the liquid culture in BHI medium supplemented with bleomycin and cultured by shaking (140 rpm) for 72 hours at 26°C. The resulting culture was used to inoculate 2 cultures: 100 ml expressing in BHI medium, supplemented with bleomycin and tetracycline (100 µg / ml) and 10 ml control culture in BHI medium, supplemented with bleomycin, followed by shaking (140 rpm) for 72 hours at 26°C. At 24-hour intervals, 100 µl samples were taken of each culture, applied to a 96-well plate by measuring OD optical density in a TECAN Infinity M200Pro plate reader at 600 nm wavelength and blecherry protein fluorescence at an excitation wavelength of 590 nm and an emission wavelength of 620 nm. In the exemplary embodiment shown in Figures 8, 9 and 10, the increase in the expression level of the blecherry was correlated with the OD growth of the culture. Above the 48-hour cultivation time, the OD600 level and blecherry expression did not increase significantly, hence the cultures were stopped after 72 hours and *Leishmania tarentolae* T7-TR recombinant cells were centrifuged at 4,000 g for 20 min. The expressed BChE as proteins secreted outside the cell remained in the medium. Centrifuged cells contained a blecherry reporter protein due to its cytoplasmic location, thus the expressed blecherry and BChE proteins were separated. Figure 8 shows the dependence of OD₆₀₀ from the time of cultivation after induction, Figure 9 shows the dependence of blecherry fluorescence from the time of cultivation after induction, Figure 10 shows the centrifuged cells after 72 hours of culture after induction, where the tetracycline-induced culture shows a distinct pink colour (on the right-hand side of the photograph) compared to a control, uninduced (on the left-hand side of the photograph).

Example 8. Isolation of recombinant BChE proteins.

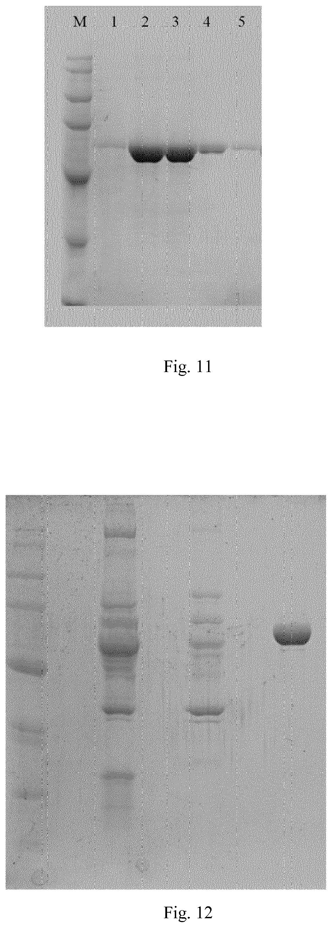

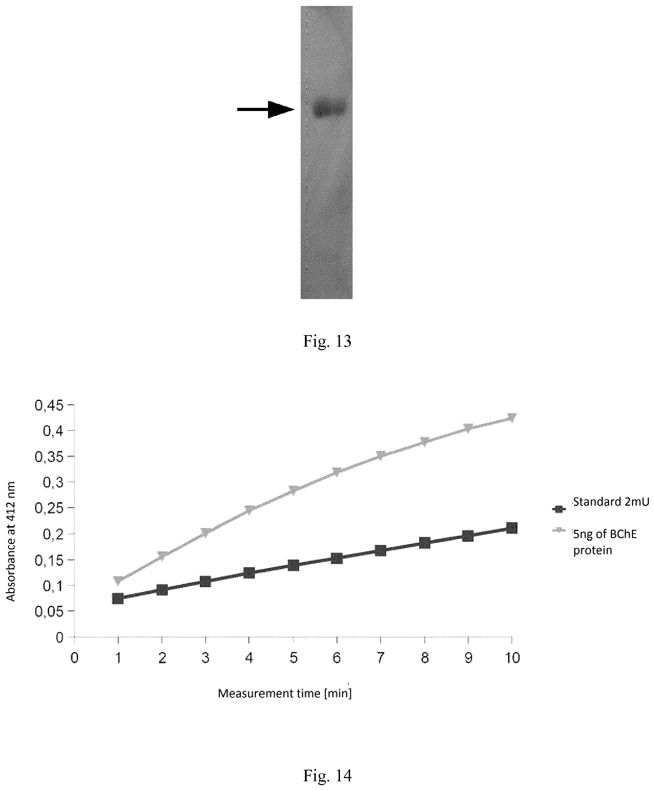

In the exemplary embodiment presented in Figures 11, 12 and 13, the results of the isolation procedure of the recombinant truncated version of BChE were presented, as outlined in Examples 1,2,3,4,6,7 and Seq.2. Identical procedure was used for the version of the BChE holoenzyme indicated in Examples 1,3,5,6,7 and Seq.5. In an exemplary embodiment, a laboratory scale preparation was made with 100 ml of a supernatant from tetracycline-induced *Leishmania tarentolae* T7-TR cell culture transformed with BChE expression cassettes. The procedure is directly scalable to the industrial process, after the use of bioreactors, centrifuges and chromatographic systems with the appropriate volume scaling required. 100 ml of the centrifuged culture supernatant was filtered through a microbial filter with a pore size of 0.22 µm to remove *Leishmania tarentolae* T7-TR cell debris and fragments thereof. To the filtrate, cooled to 4°C, crystalline ammonium sulphate was added to obtain a saturated solution, precipitating the proteins contained in the filtrate. The suspension was centrifuged at 20,000 g for 30 min at 4°C, the supernatant discarded, and a precipitate of precipitated proteins was dissolved in 10 ml of buffer A for metal affinity chromatography: 50 mM NaH₂PO₄, 300 mM NaCl, 10 mM imidazole, pH 8.0. The proteins dissolved in buffer were applied to a 4 ml bed containing chelated nickel (II) ions (Sigma, USA). The column, mounted in a Hitachi-Merck chromatography system, was washed with 20 ml of buffer A, then washed with 20 ml of buffer B for metal affinity chromatography (50 mM NaH₂PO₄, 300 mM NaCl, 20 mM imidazole, pH 8.0.), containing an increased concentration of the eluent - imidazole, to elute weakly bound contaminating proteins not bearing the 6-residue His-tag. The level of contamination rinsing and subsequent BChE elution were monitored by an on-line UV monitor installed in the on-line chromatographic system at a wavelength of 280 nm. After reaching a low and stable absorbance at 280 nm, the recombinant BChE bound to the chromatographic bed was eluted with buffer C: 50 mM NaH₂PO₄, 300 mM NaCl, 500 mM imidazole, pH 8.0. 1 ml fractions were collected and analysed by SDS-PAGE denaturing electrophoresis in an 8% polyacrylamide gel. The gel was stained with Commassie Brilliant Blue, observing in the elution fractions (paths 2-5) coloured protein bands corresponding to the size expected for the truncated version of recombinant BChE, as indicated in Examples 2, 4, 6 and 7. In the electropherogram in Figure 11 the M path contains standards of the molecular mass of Perfect Protein Ladder proteins (EURx, Poland), path 1, leakage from the column during rinsing with buffer B. Fig. 11 shows the analysis of recombinant BChE fraction from a chromatographic bed containing chelated nickel (II) ions. A single protein band is evidently observed, dominant electropherogram in paths 2 and 3, containing highly purified, homogeneous, recombinant BChE, indicated on Seq.2. Analogous comparative experiments were performed using clones containing full-length BChE genes, containing the natural horse secretory signals indicated in Example 3 and in Fig. 3. The obtained expression levels allowed detection of BChE enzymatic activity by Ellman's functional test, but it was several dozen times lower compared to the expression of the shortened version of BChE bearing the *Leishmania* secretory signal, which indicates the important role of the use of the *Leishmania* secretion signal in the present invention. Fig. 12 depicts a comparison of the purity of the resulting BChE preparation to the purity of commercially available BChE formulations: in the M pathway, the molecular weight standards of the Perfect Protein Ladder proteins were visualized, in the path 1 the BChE preparation from Sigma-Aldrich (USA) was visualized, in the path 2 the BChE from Biomed Lublin (Poland) was visualized. The unprecedented purity of the recombinant BChE formulation obtained in the exemplary embodiment in Example 8 is evident. To ultimately validate the identity of the resulting protein preparation with the recombinant BChE designed, synthesized, cloned and expressed in Examples 2, 4, and 7, a Western blotting analysis was carried out, electro-transferring proteins from an 8% SDS-PAGE gel to a PVDF membrane for 60 min at a current of 100 mA in transfer buffer: 25 mM Tris-HCl, 192 mM glycine, 10% metanol, 0,1% SDS. The nitrocellulose membrane was then rinsed in TBS buffer (50 mM Tris-HCl pH 7.5, 0.5 M NaCl) and blocked for 16 hours in TBS buffer with 3% non-fat milk at 4°C. The nitrocellulose membrane was then incubated with commercial rabbit polyclonal antibodies (Santa Cruz Technology, USA) using a 1:500 dilution for 1 hour and washed three times in TBS buffer. Immunodetection of the bound primary antibodies was done using 1-hour incubation with the secondary anti-rabbit antibodies (1: 2000 dilution) coupled to the alkaline phosphatase, followed by colour reaction with the substrate solution of: nitro-blue tetrazolium salt (NBT) and 5-bromo 4- chloro-3-indyl phosphate (BCIP), giving a deep blue colour at the binding site of conjugated antibodies. In the exemplary embodiment shown in Fig. 13, a specific immunodetection signal is seen in the expected migration position of the recombinant band, of fusion protein GA-BChE-His (marked with an arrow) in an 8% SDS-PAGE gel.

Example 9. Functional enzyme tests of recombinant BChEs.

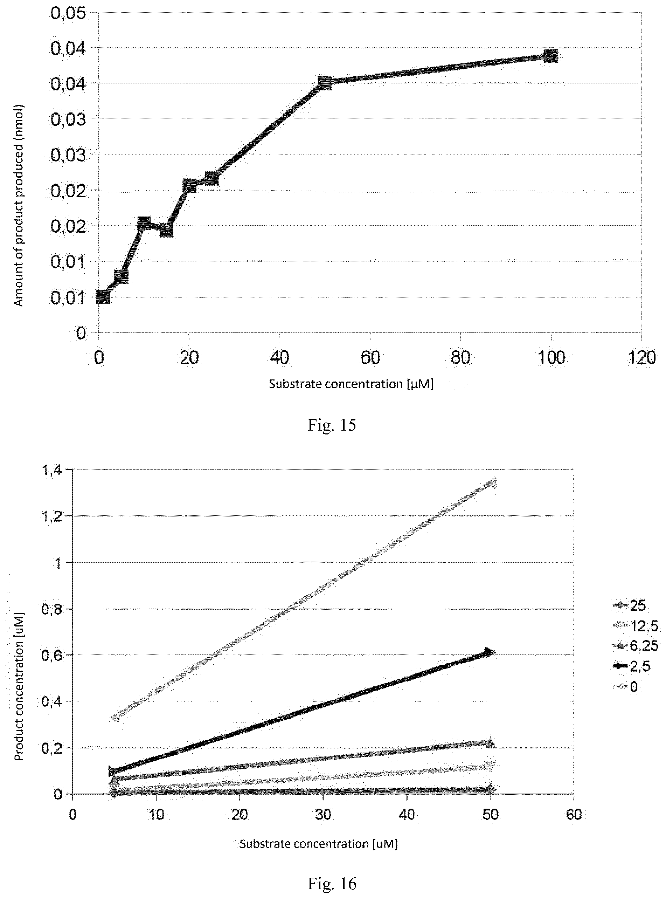

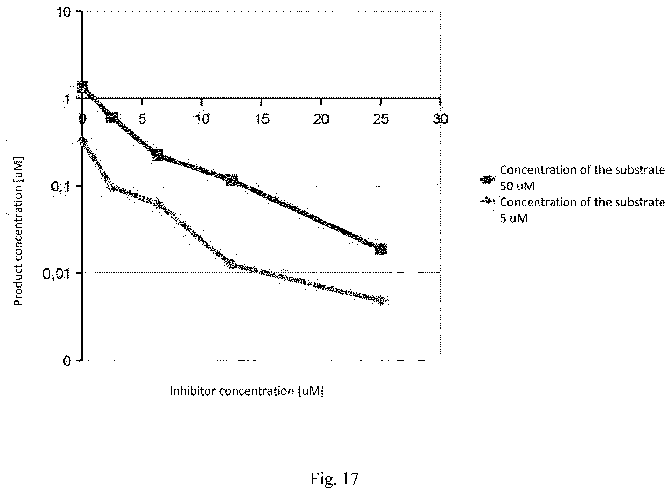

A recombinant GA-BChE-His enzyme, obtained in the exemplified embodiment shown in Figures 2, 4, 6, 7, 8, 9, 10, 11, 12, 13 and indicated in Examples 2, 4, 6, 7 and 8, and on Seq.2 were subjected to functional enzymatic tests. Functional enzyme tests of the recombinant BChE indicated in Examples 1,2,3,4,6,7,8 are shown. The obtained preparation of the recombinant shortened version of BChE at the concentration of 5 mg / ml was subjected to the analysis of enzymatic activity using the Ellman's method. The method is based on the measurement of a coloured reaction product that results from the hydrolysis of S-butyrylthiocholine iodide by BChE to the thiocholine, which in turn reacts with DTNB (5,5'-dithiobis-2-nitrobenzoic acid), producing a coloured product. Maximum absorbance is at a wavelength of 412 nm. Considering the molar absorbance coefficient of a coloured product, concentration of which is equal to the concentration of the substrate decomposed by BChE, the enzymatic activity of BChE can be quantified. In the exemplary embodiment presented in Figures 14, 15, 16 and 17, measurements of BChE protein activity were carried out on 96-well plates in a TECAN Infinity M200Pro plate reader, measuring absorbance at 412 nm every 1 min. The results for the GA-BChE-His protein preparation and also for the standard from the Arbor Assay kit (USA) indicate enzymatic activity of the purified recombinant GA-BChE-His protein preparation at a high level of 270 U / mg. In addition, a detailed enzymatic characterization was performed by plotting the Michaelis-Menten curve, shown in Fig. 15, from which the Michaelis constant (Km) of 20 µM was determined for S-butyrylthiocholine iodide. The enzymatic reactions were carried out at 25°C in 100 mM in phosphate buffer at pH 7.4. The vast majority of specific inhibitors described and used in the work on the enzymatic activity characteristics of ChE and BChE are highly toxic, some of them are classified as CWAs, requiring work in certified laboratories. Therefore, in order to verify the substrate specificity of the recombinant BChE obtained, the effect of a BChE inhibitor described in the literature, with a relatively low toxicity - methylene blue, was examined. In the exemplary embodiment presented in Fig. 16, BChE activity measurements were performed in the presence of different concentrations of methylene blue. The reaction catalysed by BChE is inhibited as the concentration of methylene blue increases. In each measurement 10 ng of purified protein and methylene blue in the following concentrations were used: 25 um, 12,5 mM, 6,25 mM, 2,5 mM, 0 mM. The results obtained confirmed the inhibitory effect of methylene blue, thereby additionally confirming that the cloned and expressed in *Leishmania tarentolae* T7-TR protein is actually a functional GA-BChE-His enzyme, indicated on Seq.2, exhibiting activity described in the BChE literature. ChE and BChE enzymes, cloned and expressed in the microorganism *Leishmania tarantolae* can find a number of current and future applications: as an antidote, for example, but not exclusively, to exposure to chemical warfare agents, pesticides, other poisons, venoms of snake, fish, other vertebrates, insects, other invertebrates, drugs, medicines; as medicines or substances for drug testing (e.g., BChE and CHE inhibitors) intended for the treatment of neurodegenerative diseases, for example, but not exclusively Alzheimer's disease, dementia, alcoholism, drug addiction, traumatic injury of the nervous system; as components of biosensors detecting, for example, but not limited to, CWAs, pesticides, industrial poisons, drugs.
## Claims

1. A DNA vector for transformation and integration with *Leishmania tarentolae* chromosome containing a gene according to Seq.3 or Seq.7 or Seq.8 encoding a truncated version of equine BChE, devoid of the tetrameric domain, the vector being for biosynthesis of a recombinant protein from the Open Reading Frame contained in the gene, wherein gene contains the Open Reading Frame encoding a protein with Seq.1 containing 564 amino acid residues and bearing at an N-terminus a secreted *Leishmania sequence* with 23 amino acid residues and additionally containing 2 Gly-Ala amino acid residues, and at the C-terminus containing 4 Leu-Lys-Gly-Thr amino acid residues and a 6-residue His-tag.

2. Expressive DNA vector for transformation and integration with *Leishmania tarentolae* chromosome, for the production of a protein with Seq.4, containing a total of 614 amino acid residues, including the additional 2 amino acid residues Met-Ala at the N-terminus, and with an additional 4 Leu-Lys-Gly-Thr amino acid residues and the 6-residue His-tag at the C-terminus, wherein the vector comprises a gene according to Seq.6 encoding equine BChE holoenzyme

3. DNA vector for transformation and integration with *Leishmania tarentolae* chromosome, for the production of protein with Seq.4 containing a total of 614 amino acid residues, including the additional 2 amino acid residues Met-Ala at the N-terminus and bearing additional 4 amino acid residues Leu -Lys-Gly-Thr and 6-residue His-tag at the C-terminus, wherein the vector comprises a synthetic gene with a sequence of Seq.3 encoding the equine BChE holoenzyme..

4. The DNA vector according to the claim 1 or claim 2, wherein the vector is a shuttle vector for cloning and expression of protein, further comprising a minimum of two origin of replication, preferably for *Escherichia coli* and / or *Leishmania tarentolae.*

5. The DNA vector according to claim 1 or claim 2 , wherein the vector comprises a secretory signal coding sequence, preferably *Leishmania* in origin, antibiotic resistance gene, preferably encoding bleomycin and / or ampicillin, transcription promoter, preferably T7 bacteriophage, transcription repressor gene, preferably regulated by tetracycline, operator gene, preferably regulated by a tetracycline repressor, a translation initiation signal, a coding sequence for a translation stop signal, preferably a sequence encoding 6 histidine residues.

## Patentansprüche

1. Ein DNA-Vektor zur Transformation und Integration mit einem Chromosom von *Leishmania tarentolae,* das ein Gen gemäß Sequenz 3 oder Sequenz 7 oder Sequenz 8 enthält, das eine abgeschnittene Version vom Pferde-BChE ohne die tetramerische Domäne kodiert, wobei der Vektor für die Biosynthese eines rekombinanten Proteins aus dem im Gen enthaltenen offenen Leserahmen bestimmt ist, wobei das Gen den offenen Leserahmen enthält, der für ein Protein mit der Sequenz 1 enthält, das 564 Aminosäurereste enthält und an einem N-Terminus eine sekretierte *Leishmania-Sequenz* mit 23 Aminosäureresten trägt und zusätzlich 2 Gly-Ala-Aminosäurereste enthält, und am C-Terminus 4 Leu-Lys-Gly-Thr-Aminosäurereste und einen His-Tag mit 6 Resten enthält.

2. Ein expressiver DNA-Vektor zur Transformation und Integration mit einem Chromosom von *Leishmania tarentolae* zur Bildung eines Proteins mit Sequenz 4, der insgesamt 614 Aminosäurereste, einschließlich der zusätzlichen 2 Aminosäurereste Met-Ala am N-Terminus und zusätzlichen 4 Leu-Lys-Gly-Thr-Aminosäurereste und den His-Tag mit 6 Resten am C-Terminus enthält, wobei der Vektor ein Gen gemäß Sequenz 6 enthält, das für das Pferde-BChE-Holoenzym kodiert.

3. Ein DNA-Vektor zur Transformation und Integration mit einem Chromosom von *Leishmania tarentolae* zur Bildung eines Proteins mit der Sequenz 4, das insgesamt 614 Aminosäurereste enthält, einschließlich der zusätzlichen 2 Aminosäurereste Met-Ala am N-Terminus, und das zusätzliche 4 Aminosäurereste Leu-Lys-Gly-Thr und einen His-Tag mit 6 Resten am C-Terminus enthält, wobei der Vektor ein synthetisches Gen mit der Sequenz 3 enthält, die das Pferde-BChE-Holoenzym kodiert.

4. Ein DNA-Vektor nach Anspruch 1 oder Anspruch 2, wobei der Vektor ein Shuttle-Vektor zum Klonen und Expression von Proteinen ist, der ferner mindestens zwei Replikationsausgangspunkte erhält, vorzugsweise für *Escherichia coli* und/oder *Leishmania tarentolae.*

5. Ein DNA-Vektor nach Anspruch 1 oder Anspruch 2, wobei der Vektor eine für ein sekretorisches Signal kodierende Sequenz, vorzugsweise *Leishmania* im Stamm, ein Antibiotikaresistenzgen umfasst, das vorzugsweise Bleomycin und/oder Ampicillin kodiert, einen Transkriptionspromotor, vorzugsweise einen T7-Bakteriophagen, ein Transkriptionsrepressor-Gen, das vorzugsweise durch Tetracyclin reguliert wird, ein Operator-Gen, das vorzugsweise durch einen Tetracyclin-Repressor reguliert wird, ein Translation initiierendes Signal, eine kodierende Sequenz für ein Translation unterbrechendes Signal, vorzugsweise eine Sequenz, die 6 Histidinreste kodiert, umfasst.

## Revendications

1. Vecteur d'ADN pour la transformation et l'intégration avec le chromosome de *Leishmania tarentolae* contenant un gène selon Seq.3 ou Seq.7 ou Seq.8 codant pour une version tronquée de la BChE équine, dépourvue du domaine tétramérique, le vecteur étant destiné à la biosynthèse d'une protéine recombinante du cadre de lecture ouvert contenu dans le gène, dans lequel le gène contient le cadre de lecture ouvert codant pour une protéine avec Seq.1 contenant 564 résidus d'acides aminés et portant à une extrémité N une séquence de *Leishmania* sécrétée avec 23 résidus d'acides aminés et contenant en plus 2 résidus d'acides aminés Gly-Ala, et au C -terminal contenant 4 résidus d'acides aminés Leu-Lys-Gly-Thr et un marqueur His à 6 résidus.

2. Vecteur d'ADN expressif pour la transformation et l'intégration avec le chromosome de *Leishmania tarentolae,* pour la production d'une protéine avec Seq.4, contenant un total de 614 résidus d'acides aminés, comprenant les 2 résidus d'acides aminés Met-Ala supplémentaires à l'extrémité N-terminale, et avec 4 résidus d'acides aminés supplémentaires Leu-Lys-Gly-Thr et le marqueur His à 6 résidus à l'extrémité C-terminale, dans lequel le vecteur comprend un gène selon Seq.6 codant pour l'holoenzyme BChE équine.

3. Vecteur d'ADN pour la transformation et l'intégration avec le chromosome de *Leishmania tarentolae,* pour la production de protéine avec Seq.4 contenant un total de 614 résidus d'acides aminés, y compris les 2 résidus d'acides aminés supplémentaires Met-Ala à l'extrémité N-terminale et portant 4 acides aminés supplémentaires résidus Leu-Lys-Gly-Thr et His-tag à 6 résidus à l'extrémité C-terminale, dans lequel le vecteur comprend un gène synthétique avec une séquence de Seq.3 codant pour l'holoenzyme BChE équine.

4. Vecteur d'ADN selon la revendication 1 ou la revendication 2, dans lequel le vecteur est un vecteur navette pour le clonage et l'expression de protéine, comprenant en outre un minimum de deux origines de réplication, de préférence pour *Escherichia coli* et/ou *Leishmania tarentolae.*

5. Vecteur d'ADN selon la revendication 1 ou la revendication 2, dans lequel le vecteur comprend une séquence sécrétoire codant pour le signal, de préférence d'origine *Leishmania,* gène de résistance aux antibiotiques, de préférence codant pour la bléomycine et/ou l'ampicilline, promoteur de transcription, de préférence bactériophage T7, gène répresseur de transcription, de préférence régulé par la tétracycline, gène opérateur, de préférence régulé par un répresseur tétracycline, un signal d'initiation de la traduction, une séquence codant pour un signal d'arrêt de la traduction, de préférence une séquence codant pour 6 résidus histidine.


<110> University of Gdansk
<120> Geny kodujacy butyrylocholinoesterazê, wektory DNA do uzyskiwania
   rekombinantowej, biologicznie aktywnej butyrylocholinoesterazy konskiej
   lub acetylocholinoesterazy i jej pochodnych oraz sposob uzyskiwania
   biologicznie aktywnych butyrylocholinoestaraz i cholinoesteraz
<160> 11
<210> 1
   <211> PRT
   <212> 564
   <213> Leishmania tarentolae
<400> 1 
Met Ala Ser Arg Leu Val Arg Val Leu Ala Ala Met Leu Val Ala
1               5                   10                  15

Ala Ala Val Ser Val Asp Ala Gly Ala Glu Glu Asp Ile Ile Ile Thr
            20                  25                  30

Thr Lys Asn Gly Lys Val Arg Gly Met Asn Leu Pro Val Leu Gly Gly
        35                  40                  45

Thr Val Thr Ala Phe Leu Gly Ile Pro Tyr Ala Gln Pro Pro Leu Gly
    50                  55                  60

Arg Leu Arg Phe Lys Lys Pro Gln Ser Leu Thr Lys Trp Ser Asn Ile
65                  70                  75                  80

Trp Asn Ala Thr Lys Tyr Ala Asn Ser Cys Tyr Gln Asn Thr Asp Gln
                85                  90                  95

Ser Phe Pro Gly Phe Leu Gly Ser Glu Met Trp Asn Pro Asn Thr Glu
            100                 105                 110

Leu Ser Glu Asp Cys Leu Tyr Leu Asn Val Trp Ile Pro Ala Pro Lys
        115                 120                 125

Pro Lys Asn Ala Thr Val Met Ile Trp Ile Tyr Gly Gly Gly Phe Gln
    130                 135                 140

Thr Gly Thr Ser Ser Leu Pro Val Tyr Asp Gly Lys Phe Leu Ala Arg
145                 150                 155                 160

Val Glu Arg Val Ile Val Val Ser Met Asn Tyr Arg Val Gly Ala Leu
                165                 170                 175

Gly Phe Leu Ala Leu Ser Glu Asn Pro Glu Ala Pro Gly Asn Met Gly
            180                 185                 190

Leu Phe Asp Gln Gln Leu Ala Leu Gln Trp Val Gln Lys Asn Ile Ala
        195                 200                 205

Ala Phe Gly Gly Asn Pro Arg Ser Val Thr Leu Phe Gly Glu Ser Ala
    210                 215                 220

Gly Ala Ala Ser Val Ser Leu His Leu Leu Ser Pro Arg Ser Gln Pro
 
225                 230                 235                 240

Leu Phe Thr Arg Ala Ile Leu Gln Ser Gly Ser Ser Asn Ala Pro Trp
                245                 250                 255

Ala Val Thr Ser Leu Tyr Glu Ala Arg Asn Arg Thr Leu Thr Leu Ala
            260                 265                 270

Lys Arg Met Gly Cys Ser Arg Asp Asn Glu Thr Glu Met Ile Lys Cys
        275                 280                 285

Leu Arg Asp Lys Asp Pro Gln Glu Ile Leu Leu Asn Glu Val Phe Val
    290                 295                 300

Val Pro Tyr Asp Thr Leu Leu Ser Val Asn Phe Gly Pro Thr Val Asp
305                 310                 315                 320

Gly Asp Phe Leu Thr Asp Met Pro Asp Thr Leu Leu Gln Leu Gly Gln
                325                 330                 335

Phe Lys Arg Thr Gln Ile Leu Val Gly Val Asn Lys Asp Glu Gly Thr
            340                 345                 350

Ala Phe Leu Val Tyr Gly Ala Pro Gly Phe Ser Lys Asp Asn Asn Ser
        355                 360                 365

Ile Ile Thr Arg Lys Glu Phe Gln Glu Gly Leu Lys Ile Phe Phe Pro
    370                 375                 380

Arg Val Ser Glu Phe Gly Arg Glu Ser Ile Leu Phe His Tyr Met Asp
385                 390                 395                 400

Trp Leu Asp Asp Gln Arg Ala Glu Asn Tyr Arg Glu Ala Leu Asp Asp
                405                 410                 415

Val Val Gly Asp Tyr Asn Ile Ile Cys Pro Ala Leu Glu Phe Thr Lys
            420                 425                 430

Lys Phe Ser Glu Leu Gly Asn Asp Ala Phe Phe Tyr Tyr Phe Glu His
        435                 440                 445

Arg Ser Thr Lys Leu Pro Trp Pro Glu Trp Met Gly Val Met His Gly
    450                 455                 460

Tyr Glu Ile Glu Phe Val Phe Gly Leu Pro Leu Glu Arg Arg Val Asn
465                 470                 475                 480

Tyr Thr Lys Ala Glu Glu Ile Leu Ser Arg Ser Ile Met Lys Arg Trp
                485                 490                 495

Ala Asn Phe Ala Lys Tyr Gly Asn Pro Asn Gly Thr Gln Ser Asn Ser
            500                 505                 510

Thr Arg Trp Pro Val Phe Lys Ser Thr Glu Gln Lys Tyr Leu Thr Leu
        515                 520                 525

Asn Thr Glu Ser Pro Lys Val Tyr Thr Lys Leu Arg Ala Gln Gln Cys
    530                 535                 540

Arg Phe Trp Thr Leu Phe Phe Pro Lys Val Leu Lys Gly Thr His His
545                 550                 555                 560

His His His His
<210> 2
   <211> PRT
   <212> 541
   <213> Leishmania tarentolae
<400> 1 
Gly Ala Glu Glu Asp Ile Ile Ile Thr Thr Lys Asn Gly Lys Val Arg
1               5                   10                  15

Gly Met Asn Leu Pro Val Leu Gly Gly Thr Val Thr Ala Phe Leu Gly
            20                  25                  30

Ile Pro Tyr Ala Gln Pro Pro Leu Gly Arg Leu Arg Phe Lys Lys Pro
        35                  40                  45

Gln Ser Leu Thr Lys Trp Ser Asn Ile Trp Asn Ala Thr Lys Tyr Ala
    50                  55                  60

Asn Ser Cys Tyr Gln Asn Thr Asp Gln Ser Phe Pro Gly Phe Leu Gly
65                  70                  75                  80

Ser Glu Met Trp Asn Pro Asn Thr Glu Leu Ser Glu Asp Cys Leu Tyr
                85                  90                  95

Leu Asn Val Trp Ile Pro Ala Pro Lys Pro Lys Asn Ala Thr Val Met
            100                 105                 110

Ile Trp Ile Tyr Gly Gly Gly Phe Gln Thr Gly Thr Ser Ser Leu Pro
        115                 120                 125

Val Tyr Asp Gly Lys Phe Leu Ala Arg Val Glu Arg Val Ile Val Val
    130                 135                 140

Ser Met Asn Tyr Arg Val Gly Ala Leu Gly Phe Leu Ala Leu Ser Glu
145                 150                 155                 160

Asn Pro Glu Ala Pro Gly Asn Met Gly Leu Phe Asp Gln Gln Leu Ala
                165                 170                 175

Leu Gln Trp Val Gln Lys Asn Ile Ala Ala Phe Gly Gly Asn Pro Arg
            180                 185                 190

Ser Val Thr Leu Phe Gly Glu Ser Ala Gly Ala Ala Ser Val Ser Leu
        195                 200                 205

His Leu Leu Ser Pro Arg Ser Gln Pro Leu Phe Thr Arg Ala Ile Leu
    210                 215                 220

Gln Ser Gly Ser Ser Asn Ala Pro Trp Ala Val Thr Ser Leu Tyr Glu
225                 230                 235                 240

Ala Arg Asn Arg Thr Leu Thr Leu Ala Lys Arg Met Gly Cys Ser Arg
                245                 250                 255

Asp Asn Glu Thr Glu Met Ile Lys Cys Leu Arg Asp Lys Asp Pro Gln
            260                 265                 270

Glu Ile Leu Leu Asn Glu Val Phe Val Val Pro Tyr Asp Thr Leu Leu
        275                 280                 285

Ser Val Asn Phe Gly Pro Thr Val Asp Gly Asp Phe Leu Thr Asp Met
 
290                 295                 300

Pro Asp Thr Leu Leu Gln Leu Gly Gln Phe Lys Arg Thr Gln Ile Leu
305                 310                 315                 320

Val Gly Val Asn Lys Asp Glu Gly Thr Ala Phe Leu Val Tyr Gly Ala
                325                 330                 335

Pro Gly Phe Ser Lys Asp Asn Asn Ser Ile Ile Thr Arg Lys Glu Phe
            340                 345                 350

Gln Glu Gly Met Lys Ile Phe Phe Pro Arg Val Ser Glu Phe Gly Arg
        355                 360                 365

Glu Ser Ile Leu Phe His Tyr Met Asp Trp Leu Asp Asp Gln Arg Ala
    370                 375                 380

Glu Asn Tyr Arg Glu Ala Leu Asp Asp Val Val Gly Asp Tyr Asn Ile
385                 390                 395                 400

Ile Cys Pro Ala Leu Glu Phe Thr Lys Lys Phe Ser Glu Leu Gly Asn
                405                 410                 415

Asp Ala Phe Phe Tyr Tyr Phe Glu His Arg Ser Thr Lys Leu Pro Trp
            420                 425                 430

Pro Glu Trp Met Gly Val Met His Gly Tyr Glu Ile Glu Phe Val Phe
        435                 440                 445

Gly Leu Pro Leu Glu Arg Arg Val Asn Tyr Thr Lys Ala Glu Glu Ile
    450                 455                 460

Leu Ser Arg Ser Ile Met Lys Arg Trp Ala Asn Phe Ala Lys Tyr Gly
465                 470                 475                 480

Asn Pro Asn Gly Thr Gln Ser Asn Ser Thr Arg Trp Pro Val Phe Lys
                485                 490                 495

Ser Thr Glu Gln Lys Tyr Leu Thr Leu Asn Thr Glu Ser Pro Lys Val
            500                 505                 510

Tyr Thr Lys Leu Arg Ala Gln Gln Cys Arg Phe Trp Thr Leu Phe Phe
        515                 520                 525

Pro Lys Val Leu Lys Gly Thr His His His His His His
    530                 535                 540
<210> 3
   <211> DNA
   <212> 1587
   <213> Leishmania tarentolae
<400> 1 
gaagaagaca tcatcatcac caccaaaaac ggtaaagttc gtggtatgaa
cctgccggtt 60
ctgggtggta ccgttaccgc tttcctgggt atcccgtacg ctcagccgcc
gctgggtcgt 120
ctgcgtttca aaaaaccgca gtccctgacc aaatggtcca acatctggaa
cgctaccaaa 180
tacgctaact cctgctacca gaacaccgac cagtccttcc cgggtttcct
gggttccgaa 240
atgtggaacc cgaacaccga actgtccgaa gactgcctgt acctgaacgt
 
ttggatcccg 300
gctccgaaac cgaaaaacgc taccgttatg atctggatct acggtggtgg
tttccagacc 360
ggtacctcct ccctgccggt ttacgacggt aaattcctgg ctcgtgttga
acgtgttatc 420
gttgtttcca tgaactaccg tgttggtgct ctgggtttcc tggctctgtc
cgaaaacccg 480
gaagctccgg gtaacatggg tctgttcgac cagcagctgg ctctgcagtg
ggttcagaaa 540
aacatcgctg ctttcggtgg taacccgcgt tccgttaccc tgttcggtga
atccgctggt 600
gctgcttccg tttccctgca cctgctgtcc ccgcgttccc agccgctgtt
cacccgtgct 660
atcctgcagt ccggttcctc caacgctccg tgggctgtta cctccctgta
cgaagctcgt 720
aaccgtaccc tgaccctggc taaacgtatg ggttgctccc gtgacaacga
aaccgaaatg 780
atcaaatgcc tgcgtgacaa agacccgcag gaaatcctgc tgaacgaagt
tttcgttgtt 840
ccgtacgaca ccctgctgtc cgttaacttc ggtccgaccg ttgacggtga
cttcctgacc 900
gacatgccgg acaccctgct gcagctgggt cagttcaaac gtacccagat
cctggttggt 960
gttaacaaag acgaaggtac cgctttcctg gtttacggtg ctccgggttt
ctccaaagac 1020
aacaactcca tcatcacccg taaagaattc caggaaggtc tgaaaatctt
cttcccgcgt 1080
gtttccgaat tcggtcgtga atccatcctg ttccactaca tggactggct
ggacgaccag 1140
cgtgctgaaa actaccgtga agctctggac gacgttgttg gtgactacaa
catcatctgc 1200
ccggctctgg aattcaccaa aaaattctcc gaactgggta acgacgcttt
cttctactac 1260
ttcgaacacc gttccaccaa actgccgtgg ccggaatgga tgggtgttat
gcacggttac 1320
gaaatcgaat tcgttttcgg tctgccgctg gaacgtcgtg ttaactacac
caaagctgaa 1380
gaaatcctgt cccgttccat catgaaacgt tgggctaact tcgctaaata
cggtaacccg 1440
aacggtaccc agtccaactc cacccgttgg ccggttttca aatccaccga
acagaaatac 1500
ctgaccctga acaccgaatc cccgaaagtt tacaccaaac tgcgtgctca
gcagtgccgt 1560
ttctggaccc tgttcttccc gaaagtt
1587
<210> 4
   <211> PRT
   <212> 614
   <213> Leishmania tarentolae
<400> 1 
Met Ala Met Gln Ser Trp Gly Thr Ile Ile Cys Ile Arg Ile Leu Leu
1               5                   10                  15

Arg Phe Leu Leu Leu Trp Val Leu Ile Gly Asn Ser His Thr Glu Glu
            20                  25                  30

Asp Ile Ile Ile Thr Thr Lys Asn Gly Lys Val Arg Gly Met Asn Leu
        35                  40                  45
 
Pro Val Leu Gly Gly Thr Val Thr Ala Phe Leu Gly Ile Pro Tyr Ala
    50                  55                  60

Gln Pro Pro Leu Gly Arg Leu Arg Phe Lys Lys Pro Gln Ser Leu Thr
65                  70                  75                  80

Lys Trp Ser Asn Ile Trp Asn Ala Thr Lys Tyr Ala Asn Ser Cys Tyr
                85                  90                  95

Gln Asn Thr Asp Gln Ser Phe Pro Gly Phe Leu Gly Ser Glu Met Trp
            100                 105                 110

Asn Pro Asn Thr Glu Leu Ser Glu Asp Cys Leu Tyr Leu Asn Val Trp
        115                 120                 125

Ile Pro Ala Pro Lys Pro Lys Asn Ala Thr Val Met Ile Trp Ile Tyr
    130                 135                 140

Gly Gly Gly Phe Gln Thr Gly Thr Ser Ser Leu Pro Val Tyr Asp Gly
145                 150                 155                 160

Lys Phe Leu Ala Arg Val Glu Arg Val Ile Val Val Ser Met Asn Tyr
                165                 170                 175

Arg Val Gly Ala Leu Gly Phe Leu Ala Leu Ser Glu Asn Pro Glu Ala
            180                 185                 190

Pro Gly Asn Met Gly Leu Phe Asp Gln Gln Leu Ala Leu Gln Trp Val
        195                 200                 205

Gln Lys Asn Ile Ala Ala Phe Gly Gly Asn Pro Arg Ser Val Thr Leu
    210                 215                 220

Phe Gly Glu Ser Ala Gly Ala Ala Ser Val Ser Leu His Leu Leu Ser
225                 230                 235                 240

Pro Arg Ser Gln Pro Leu Phe Thr Arg Ala Ile Leu Gln Ser Gly Ser
                245                 250                 255

Ser Asn Ala Pro Trp Ala Val Thr Ser Leu Tyr Glu Ala Arg Asn Arg
            260                 265                 270

Thr Leu Thr Leu Ala Lys Arg Met Gly Cys Ser Arg Asp Asn Glu Thr
        275                 280                 285

Glu Met Ile Lys Cys Leu Arg Asp Lys Asp Pro Gln Glu Ile Leu Leu
    290                 295                 300

Asn Glu Val Phe Val Val Pro Tyr Asp Thr Leu Leu Ser Val Asn Phe
305                 310                 315                 320

Gly Pro Thr Val Asp Gly Asp Phe Leu Thr Asp Met Pro Asp Thr Leu
                325                 330                 335

Leu Gln Leu Gly Gln Phe Lys Arg Thr Gln Ile Leu Val Gly Val Asn
            340                 345                 350

Lys Asp Glu Gly Thr Ala Phe Leu Val Tyr Gly Ala Pro Gly Phe Ser
        355                 360                 365

Lys Asp Asn Asn Ser Ile Ile Thr Arg Lys Glu Phe Gln Glu Gly Leu
    370                 375                 380
 
Lys Ile Phe Phe Pro Arg Val Ser Glu Phe Gly Arg Glu Ser Ile Leu
385                 390                 395                 400

Phe His Tyr Met Asp Trp Leu Asp Asp Gln Arg Ala Glu Asn Tyr Arg
                405                 410                 415

Glu Ala Leu Asp Asp Val Val Gly Asp Tyr Asn Ile Ile Cys Pro Ala
            420                 425                 430

Leu Glu Phe Thr Lys Lys Phe Ser Glu Leu Gly Asn Asp Ala Phe Phe
        435                 440                 445

Tyr Tyr Phe Glu His Arg Ser Thr Lys Leu Pro Trp Pro Glu Trp Met
    450                 455                 460

Gly Val Met His Gly Tyr Glu Ile Glu Phe Val Phe Gly Leu Pro Leu
465                 470                 475                 480

Glu Arg Arg Val Asn Tyr Thr Lys Ala Glu Glu Ile Leu Ser Arg Ser
                485                 490                 495

Ile Met Lys Arg Trp Ala Asn Phe Ala Lys Tyr Gly Asn Pro Asn Gly
            500                 505                 510

Thr Gln Ser Asn Ser Thr Arg Trp Pro Val Phe Lys Ser Thr Glu Gln
        515                 520                 525

Lys Tyr Leu Thr Leu Asn Thr Glu Ser Pro Lys Val Tyr Thr Lys Leu
    530                 535                 540

Arg Ala Gln Gln Cys Arg Phe Trp Thr Leu Phe Phe Pro Lys Val Leu
545                 550                 555                 560

Glu Leu Thr Gly Asn Ile Asp Glu Ala Glu Arg Glu Trp Lys Ala Gly
                565                 570                 575

Phe His Arg Trp Asn Asn Tyr Met Met Asp Trp Lys Asn Gln Phe Asn
            580                 585                 590

Asp Tyr Thr Ser Lys Lys Glu Ser Cys Ser Asp Phe Leu Lys Gly Thr
        595                 600                 605

His His His His His His
    610
<210> 5
   <211> PRT
   <212> 584
   <213> Leishmania tarentolae
<400> 1 
Glu Glu Asp Ile Ile Ile Thr Thr Lys Asn Gly Lys Val Arg Gly Met
1               5                   10                  15

Asn Leu Pro Val Leu Gly Gly Thr Val Thr Ala Phe Leu Gly Ile Pro
            20                  25                  30

Tyr Ala Gln Pro Pro Leu Gly Arg Leu Arg Phe Lys Lys Pro Gln Ser
        35                  40                  45

Leu Thr Lys Trp Ser Asn Ile Trp Asn Ala Thr Lys Tyr Ala Asn Ser
 
50                  55                  60

Cys Tyr Gln Asn Thr Asp Gln Ser Phe Pro Gly Phe Leu Gly Ser Glu
65                  70                  75                  80

Met Trp Asn Pro Asn Thr Glu Leu Ser Glu Asp Cys Leu Tyr Leu Asn
                85                  90                  95

Val Trp Ile Pro Ala Pro Lys Pro Lys Asn Ala Thr Val Met Ile Trp
            100                 105                 110

Ile Tyr Gly Gly Gly Phe Gln Thr Gly Thr Ser Ser Leu Pro Val Tyr
        115                 120                 125

Asp Gly Lys Phe Leu Ala Arg Val Glu Arg Val Ile Val Val Ser Met
    130                 135                 140

Asn Tyr Arg Val Gly Ala Leu Gly Phe Leu Ala Leu Ser Glu Asn Pro
145                 150                 155                 160

Glu Ala Pro Gly Asn Met Gly Leu Phe Asp Gln Gln Leu Ala Leu Gln
                165                 170                 175

Trp Val Gln Lys Asn Ile Ala Ala Phe Gly Gly Asn Pro Arg Ser Val
            180                 185                 190

Thr Leu Phe Gly Glu Ser Ala Gly Ala Ala Ser Val Ser Leu His Leu
        195                 200                 205

Leu Ser Pro Arg Ser Gln Pro Leu Phe Thr Arg Ala Ile Leu Gln Ser
    210                 215                 220

Gly Ser Ser Asn Ala Pro Trp Ala Val Thr Ser Leu Tyr Glu Ala Arg
225                 230                 235                 240

Asn Arg Thr Leu Thr Leu Ala Lys Arg Met Gly Cys Ser Arg Asp Asn
                245                 250                 255

Glu Thr Glu Met Ile Lys Cys Leu Arg Asp Lys Asp Pro Gln Glu Ile
            260                 265                 270

Leu Leu Asn Glu Val Phe Val Val Pro Tyr Asp Thr Leu Leu Ser Val
        275                 280                 285

Asn Phe Gly Pro Thr Val Asp Gly Asp Phe Leu Thr Asp Met Pro Asp
    290                 295                 300

Thr Leu Leu Gln Leu Gly Gln Phe Lys Arg Thr Gln Ile Leu Val Gly
305                 310                 315                 320

Val Asn Lys Asp Glu Gly Thr Ala Phe Leu Val Tyr Gly Ala Pro Gly
                325                 330                 335

Phe Ser Lys Asp Asn Asn Ser Ile Ile Thr Arg Lys Glu Phe Gln Glu
            340                 345                 350

Gly Leu Lys Ile Phe Phe Pro Arg Val Ser Glu Phe Gly Arg Glu Ser
        355                 360                 365

Ile Leu Phe His Tyr Met Asp Trp Leu Asp Asp Gln Arg Ala Glu Asn
    370                 375                 380

Tyr Arg Glu Ala Leu Asp Asp Val Val Gly Asp Tyr Asn Ile Ile Cys
 
385                 390                 395                 400

Pro Ala Leu Glu Phe Thr Lys Lys Phe Ser Glu Leu Gly Asn Asp Ala
                405                 410                 415

Phe Phe Tyr Tyr Phe Glu His Arg Ser Thr Lys Leu Pro Trp Pro Glu
            420                 425                 430

Trp Met Gly Val Met His Gly Tyr Glu Ile Glu Phe Val Phe Gly Leu
        435                 440                 445

Pro Leu Glu Arg Arg Val Asn Tyr Thr Lys Ala Glu Glu Ile Leu Ser
    450                 455                 460

Arg Ser Ile Met Lys Arg Trp Ala Asn Phe Ala Lys Tyr Gly Asn Pro
465                 470                 475                 480

Asn Gly Thr Gln Ser Asn Ser Thr Arg Trp Pro Val Phe Lys Ser Thr
                485                 490                 495

Glu Gln Lys Tyr Leu Thr Leu Asn Thr Glu Ser Pro Lys Val Tyr Thr
            500                 505                 510

Lys Leu Arg Ala Gln Gln Cys Arg Phe Trp Thr Leu Phe Phe Pro Lys
        515                 520                 525

Val Leu Glu Leu Thr Gly Asn Ile Asp Glu Ala Glu Arg Glu Trp Lys
    530                 535                 540

Ala Gly Phe His Arg Trp Asn Asn Tyr Met Met Asp Trp Lys Asn Gly
545                 550                 555                 560

Phe Asn Asp Tyr Thr Ser Lys Lys Glu Ser Cys Ser Asp Phe Leu Lys
                565                 570                 575

Gly Thr His His His His His His
            580
<210> 6
   <211> DNA
   <212> 1826
   <213> Leishmania tarentolae
<400> 1 
tagccatggc catgcagagc tggggtacaa tcatatgcat tcgaattctc
ttgcgatttc 60
ttctgctctg ggtgcttatc gggaactcac acactgaaga agacatcata
attacaacca 120
agaacggaaa agtcagaggg atgaacttgc cagttcttgg tggcacagta
acagcctttc 180
ttgggattcc ctatgcacag ccgcctcttg gtagacttcg attcaaaaag
ccacaatcct 240
tgactaagtg gtccaatatt tggaatgcca caaaatatgc caattcttgc
tatcagaaca 300
cagatcaaag tttcccaggc ttccttggat cagagatgtg gaacccaaac
actgaactta 360
gtgaagactg tttatatctg aatgtgtgga ttccagcacc taaaccaaaa
aatgctactg 420
taatgatatg gatctatggt ggtggtttcc aaactgggac atcatctttg
cctgtttatg 480
atggcaagtt tctggctcgg gttgaaagag ttattgtagt ttcaatgaac
tatagagtgg 540
 
gtgccctagg attcttagcc ttatcagaaa atcctgaggc accagggaac
atgggcttat 600
ttgatcaaca gttggcactt cagtgggtcc aaaaaaatat agcagccttt
ggtggtaatc 660
ctagaagtgt aactctcttt ggagaaagtg caggagcagc ttcagttagc
cttcatttac 720
tttctcctag aagccagcct ttgtttacca gagccattct gcaaagtgga
tcctctaatg 780
ccccttgggc agtaacatct ctgtatgaag ctaggaacag aacattgacc
ctagctaaac 840
gtatgggttg ctctagggac aatgagactg agatgatcaa atgtcttcga
gacaaagatc 900
cccaggaaat tcttctgaat gaagtatttg ttgtccccta tgatactctc
ttgtcagtaa 960
actttggtcc aactgtggat ggcgattttc tcactgacat gccagataca
ctactccaac 1020
ttggacagtt caaaagaacc cagatcttgg tgggtgttaa taaagatgaa
gggacagcat 1080
ttttagtata tggggctcct ggtttcagca aagataacaa cagtatcata
acaagaaaag 1140
aatttcagga gggtttaaaa atattttttc caagagtgag tgagtttgga
agagaatcaa 1200
tcctttttca ttacatggac tggttagatg atcagagagc cgaaaactac
agagaggcct 1260
tggatgatgt tgttggggat tacaatatca tatgccctgc cttggagttc
accaaaaagt 1320
tctcagaatt gggaaatgat gcctttttct actattttga acaccgatcg
accaaacttc 1380
cttggccaga atggatggga gtgatgcatg gttacgaaat cgaatttgtc
tttggtttac 1440
ctctggaaag aagagttaat tacacaaaag ctgaggaaat tttgagtaga
tccattatga 1500
aacgctgggc aaattttgca aaatatggaa atccaaatgg gacccagagc
aatagcacaa 1560
gatggcctgt cttcaagagc actgaacaaa aatatttaac cttgaataca
gagtcaccaa 1620
aagtgtacac caaactacga gctcaacaat gtcgattctg gacactattt
tttcctaaag 1680
tcttggaatt gacaggaaat attgatgaag cagaacgaga atggaaagca
ggattccatc 1740
gctggaacaa ttacatgatg gactggaaaa atcaatttaa cgattacact
agcaagaaag 1800
aaagctgttc agatttctt aagagt
1826
<210> 7
   <211> DNA
   <212> 1827
   <213> Leishmania tarentolae
<400> 1 
tagccatggc catgcagtcc tggggtacca tcatctgcat ccgtatcctg
ctgcgtttcc 60
tgctgctgtg ggttctgatc ggtaactccc acaccgaaga agacatcatc
atcaccacca 120
aaaacggtaa agttcgtggt atgaacctgc cggttctggg tggtaccgtt
accgctttcc 180
tgggtatccc gtacgctcag ccgccgctgg gtcgtctgcg tttcaaaaaa
ccgcagtccc 240
tgaccaaatg gtccaacatc tggaacgcta ccaaatacgc taactcctgc
taccagaaca 300
ccgaccagtc cttcccgggt ttcctgggtt ccgaaatgtg gaacccgaac
 
accgaactgt 360
ccgaagactg cctgtacctg aacgtttgga tcccggctcc gaaaccgaaa
aacgctaccg 420
ttatgatctg gatctacggt ggtggtttcc agaccggtac ctcctccctg
ccggtttacg 480
acggtaaatt cctggctcgt gttgaacgtg ttatcgttgt ttccatgaac
taccgtgttg 540
gtgctctggg tttcctggct ctgtccgaaa acccggaagc tccgggtaac
atgggtctgt 600
tcgaccagca gctggctctg cagtgggttc agaaaaacat cgctgctttc
ggtggtaacc 660
cgcgttccgt taccctgttc ggtgaatccg ctggtgctgc ttccgtttcc
ctgcacctgc 720
tgtccccgcg ttcccagccg ctgttcaccc gtgctatcct gcagtccggt
tcctccaacg 780
ctccgtgggc tgttacctcc ctgtacgaag ctcgtaaccg taccctgacc
ctggctaaac 840
gtatgggttg ctcccgtgac aacgaaaccg aaatgatcaa atgcctgcgt
gacaaagacc 900
cgcaggaaat cctgctgaac gaagttttcg ttgttccgta cgacaccctg
ctgtccgtta 960
acttcggtcc gaccgttgac ggtgacttcc tgaccgacat gccggacacc
ctgctgcagc 1020
tgggtcagtt caaacgtacc cagatcctgg ttggtgttaa caaagacgaa
ggtaccgctt 1080
tcctggttta cggtgctccg ggtttctcca aagacaacaa ctccatcatc
acccgtaaag 1140
aattccagga aggtctgaaa atcttcttcc cgcgtgtttc cgaattcggt
cgtgaatcca 1200
tcctgttcca ctacatggac tggctggacg accagcgtgc tgaaaactac
cgtgaagctc 1260
tggacgacgt tgttggtgac tacaacatca tctgcccggc tctggaattc
accaaaaaat 1320
tctccgaact gggtaacgac gctttcttct actacttcga acaccgttcc
accaaactgc 1380
cgtggccgga atggatgggt gttatgcacg gttacgaaat cgaattcgtt
ttcggtctgc 1440
cgctggaacg tcgtgttaac tacaccaaag ctgaagaaat cctgtcccgt
tccatcatga 1500
aacgttgggc taacttcgct aaatacggta acccgaacgg tacccagtcc
aactccaccc 1560
gttggccggt tttcaaatcc accgaacaga aatacctgac cctgaacacc
gaatccccga 1620
aagtttacac caaactgcgt gctcagcagt gccgtttctg gaccctgttc
ttcccgaaag 1680
ttctggaact gaccggtaac atcgacgaag ctgaacgtga atggaaagct
ggtttccacc 1740
gttggaacaa ctacatgatg gactggaaaa accagttcaa cgactacacc
tccaaaaaag 1800
aatcctgctc cgacttcctt aagaata
1827
<210> 8
   <211> DNA
   <212> 1614
   <213> Leishmania tarentolae
<400> 1 
atagtcgacg ctggcgccga agaagacatc atcatcacca ccaaaaacgg
taaagttcgt 60
ggtatgaacc tgccggttct gggtggtacc gttaccgctt tcctgggtat
 
cccgtacgct 120
cagccgccgc tgggtcgtct gcgtttcaaa aaaccgcagt ccctgaccaa
atggtccaac 180
atctggaacg ctaccaaata cgctaactcc tgctaccaga acaccgacca
gtccttcccg 240
ggtttcctgg gttccgaaat gtggaacccg aacaccgaac tgtccgaaga
ctgcctgtac 300
ctgaacgttt ggatcccggc tccgaaaccg aaaaacgcta ccgttatgat
ctggatctac 360
ggtggtggtt tccagaccgg tacctcctcc ctgccggttt acgacggtaa
attcctggct 420
cgtgttgaac gtgttatcgt tgtttccatg aactaccgtg ttggtgctct
gggtttcctg 480
gctctgtccg aaaacccgga agctccgggt aacatgggtc tgttcgacca
gcagctggct 540
ctgcagtggg ttcagaaaaa catcgctgct ttcggtggta acccgcgttc
cgttaccctg 600
ttcggtgaat ccgctggtgc tgcttccgtt tccctgcacc tgctgtcccc
gcgttcccag 660
ccgctgttca cccgtgctat cctgcagtcc ggttcctcca acgctccgtg
ggctgttacc 720
tccctgtacg aagctcgtaa ccgtaccctg accctggcta aacgtatggg
ttgctcccgt 780
gacaacgaaa ccgaaatgat caaatgcctg cgtgacaaag acccgcagga
aatcctgctg 840
aacgaagttt tcgttgttcc gtacgacacc ctgctgtccg ttaacttcgg
tccgaccgtt 900
gacggtgact tcctgaccga catgccggac accctgctgc agctgggtca
gttcaaacgt 960
acccagatcc tggttggtgt taacaaagac gaaggtaccg ctttcctggt
ttacggtgct 1020
ccgggtttct ccaaagacaa caactccatc atcacccgta aagaattcca
ggaaggtctg 1080
aaaatcttct tcccgcgtgt ttccgaattc ggtcgtgaat ccatcctgtt
ccactacatg 1140
gactggctgg acgaccagcg tgctgaaaac taccgtgaag ctctggacga
cgttgttggt 1200
gactacaaca tcatctgccc ggctctggaa ttcaccaaaa aattctccga
actgggtaac 1260
gacgctttct tctactactt cgaacaccgt tccaccaaac tgccgtggcc
ggaatggatg 1320
ggtgttatgc acggttacga aatcgaattc gttttcggtc tgccgctgga
acgtcgtgtt 1380
aactacacca aagctgaaga aatcctgtcc cgttccatca tgaaacgttg
ggctaacttc 1440
gctaaatacg gtaacccgaa cggtacccag tccaactcca cccgttggcc
ggttttcaaa 1500
tccaccgaac agaaatacct gaccctgaac accgaatccc cgaaagttta
caccaaactg 1560
cgtgctcagc agtgccgttt ctggaccctg ttcttcccga aagttcttaa
gaat 1614
<210> 9
   <211> DNA
   <212> 8807
   <213> Leishmania tarentolae
<400> 1 
atttaaatga atacttacaa ggtactcctg cacccggcac aagcgtatgt
aattgctagc 60
acgccggtgc cgacccaacc tccaactccc gtgctcacgc gcgcacacac
gcacgcacaa 120
 
gcatcatcat catcatcatc atggtcatcc ctcccttatc tggcgaagga
ggcactgtga 180
cgcaaggttt tctgtgtctc tcctacccct ctactgattc cttctttctc
tcttcctctt 240
ctcgcactgc acccccctcc ccccccccac acacacacac ttgctgtcat
ggctgtgcgc 300
attcgctcac tcttgtcgtg tcccgtttcc ctcatggcgt cgtcgtcttc
ccctgcgcaa 360
ccacccacgc tgcacacgca cgctccacag cgcgcttcgc cttcttcgct
ctctccctag 420
cctcctctct gtcaccctcc actgccgccg cggttggccg ctctgcatcc
ttttcccgca 480
gcctttcctt ttttttttca ctgtcagtct ggctcatacc cgtggagctg
tgtctccccg 540
agcttcccat acacccaccc cccacgacct ttctcagctt cctttctcag
tcgcttgagc 600
acgcagtgca ttcacttctt cgtccctcag ttcgcctcgc tctcgctctc
tctcgcgcgc 660
gctgccgtac ccgcccctcc tcgtactccc ctccttcctc atccccaccc
cccccgtgtg 720
cgccggctcc tctctgaatt ctaatacgac tcactatagg gagatcgtcc
ctatcagtga 780
tagagatcca gtcgcagcct gaccgcatca cacatcaagg cgttacagcc
tctccgtctt 840
tgctgcaccc aagtgtattc gtgtgaccgc gttgagggat ctaatggatt
cggaactttg 900
gtcgtttggt ctctcatgct gcccaatccc atgcgctttt ctccgtgcct
ctctgtctcc 960
ccttccgttc tatttggaca acgatgtgta ctgtaatgcg tgcgccatct
gacaacgaat 1020
agatcagcag cattcgcaca cttgcacata cccagtgaag cttttgtgtc
tgtcgtattg 1080
acaacaccga ctgcaacaag gtgtagatag aagttggcct tctcgctcgc
tcgcacgctc 1140
ttcacgctcc tgctttcctt gctgtgcctt gccaccagat ctgccatggc
ctcgaggctc 1200
gtccgtgtgc tggccgccgc catgctggtt gcagcggccg tgtcggtcga
cgctggcgcc 1260
gaagaagaca tcatcatcac caccaaaaac ggtaaagttc gtggtatgaa
cctgccggtt 1320
ctgggtggta ccgttaccgc tttcctgggt atcccgtacg ctcagccgcc
gctgggtcgt 1380
ctgcgtttca aaaaaccgca gtccctgacc aaatggtcca acatctggaa
cgctaccaaa 1440
tacgctaact cctgctacca gaacaccgac cagtccttcc cgggtttcct
gggttccgaa 1500
atgtggaacc cgaacaccga actgtccgaa gactgcctgt acctgaacgt
ttggatcccg 1560
gctccgaaac cgaaaaacgc taccgttatg atctggatct acggtggtgg
tttccagacc 1620
ggtacctcct ccctgccggt ttacgacggt aaattcctgg ctcgtgttga
acgtgttatc 1680
gttgtttcca tgaactaccg tgttggtgct ctgggtttcc tggctctgtc
cgaaaacccg 1740
gaagctccgg gtaacatggg tctgttcgac cagcagctgg ctctgcagtg
ggttcagaaa 1800
aacatcgctg ctttcggtgg taacccgcgt tccgttaccc tgttcggtga
atccgctggt 1860
gctgcttccg tttccctgca cctgctgtcc ccgcgttccc agccgctgtt
cacccgtgct 1920
atcctgcagt ccggttcctc caacgctccg tgggctgtta cctccctgta
cgaagctcgt 1980
aaccgtaccc tgaccctggc taaacgtatg ggttgctccc gtgacaacga
 
aaccgaaatg 2040
atcaaatgcc tgcgtgacaa agacccgcag gaaatcctgc tgaacgaagt
tttcgttgtt 2100
ccgtacgaca ccctgctgtc cgttaacttc ggtccgaccg ttgacggtga
cttcctgacc 2160
gacatgccgg acaccctgct gcagctgggt cagttcaaac gtacccagat
cctggttggt 2220
gttaacaaag acgaaggtac cgctttcctg gtttacggtg ctccgggttt
ctccaaagac 2280
aacaactcca tcatcacccg taaagaattc caggaaggtc tgaaaatctt
cttcccgcgt 2340
gtttccgaat tcggtcgtga atccatcctg ttccactaca tggactggct
ggacgaccag 2400
cgtgctgaaa actaccgtga agctctggac gacgttgttg gtgactacaa
catcatctgc 2460
ccggctctgg aattcaccaa aaaattctcc gaactgggta acgacgcttt
cttctactac 2520
ttcgaacacc gttccaccaa actgccgtgg ccggaatgga tgggtgttat
gcacggttac 2580
gaaatcgaat tcgttttcgg tctgccgctg gaacgtcgtg ttaactacac
caaagctgaa 2640
gaaatcctgt cccgttccat catgaaacgt tgggctaact tcgctaaata
cggtaacccg 2700
aacggtaccc agtccaactc cacccgttgg ccggttttca aatccaccga
acagaaatac 2760
ctgaccctga acaccgaatc cccgaaagtt tacaccaaac tgcgtgctca
gcagtgccgt 2820
ttctggaccc tgttcttccc gaaagttctt aagggtaccc accaccatca
ccaccactag 2880
gcggccgccc tcctcctcct ttcttgttcc tttcacgtcg ccttctcggt
tgtagctggc 2940
agacgacgag tcttactttt acgtgtactt ctctatagat gatgtatgat
ctctctgcat 3000
gcgtgttcgt gcatgtgtcc gtgtgttgtg tacgcgtgcg tctcgcctca
gctctccgcg 3060
tgaaagggtt tgactgccca tgatgcgtgt gtatatccac gcgcaggcac
gcacacacac 3120
acacacacac acacacacag gcacacacag gcacacaaac gcatctcagg
ccgagccgca 3180
tacgtctctc gcacggtctc gtttatttga tcatgtagtt gagtattaaa
ttgggaagac 3240
aaaaacataa tagcacgaag agtcgggcac gaaaagcccg atctctctct
ctctctctcg 3300
tgcgcgcagg ggcgtgtggg tgcacgacga cgaggacgga gggggaagg
gaggcataaa 3360
cggatcgatc ccccatcgca catgcgggta cgagcattat ctgctgtgtc
tggtccttta 3420
tcatatcgct acccgcccgc cccccgcccc cctccccccc cccccccccg
cggctcctgt 3480
cactgtcgtt cctgcgactc cccacacacc cgcgcaacgc tcatgtcacg
aagagaaaag 3540
tagaaggcgt ggcgatgcgt tgcgcggccc cctgtccgct ggcatgcaga
cgtcgagccg 3600
tggaacggat ggtagcgaga gacaacgcgg cgatgcagga aaggagattt
actgcaggac 3660
gtctacacac gcgcgcacac cactgcgaat ggcaccgtgc tgaggaaaat
tgggggggag 3720
ggcgcacggg ggcggggcag gaaacggttg gatcagcagc actctcaact
tgtgtccgta 3780
ttgcgaagga gggagaggag cctgcccgta ttatgggcgc tgaaacgtcg
gaaccaccta 3840
tgaatcccac ttctgcgtcg cggaggcgta ctgcttccga ccacctccgc
agctcacgcg 3900
 
cgtcgagctc cctccctttc cccttctttc cgttgtgtgt gtgtgtgtgt
gtgtgtcttg 3960
tcacgatgca tggccatctc tcctccaacc ctccacgcct tcctctcccc
cgcccatcaa 4020
acgcgctacg gccacaacca tttcttcaag tatcacatcc accaccactc
ttacccacct 4080
accctcttcg actctgacac agccactgac gccctctccg cctctctcgc
tcgctgcaca 4140
ttgactctcc acagcccccc ctccctcgca cagccagccc cacccaccca
cccacccacc 4200
caccgctcta cacacctcca cagcagccgg atccaccgca tggccaagtt
gaccagtgcc 4260
gttccggtgc tcaccgcgcg cgacgtcgcc ggagcggtcg agttctggac
cgaccggctc 4320
gggttctccc gggacttcgt ggaggacgac ttcgccggtg tggtccggga
caacgtgacc 4380
ctgttcatca gcgcggtcca ggaccaggtg gtgccggaca acaccctggc
ctgggtgtgg 4440
gtgcgcggcc tggacgagct gtacgccgag tggtcggagg tcgtgtccac
gaacttccgg 4500
gacgcctccg ggccggccat gaccgagatc ggcgagcagc cgtgggggcg
ggagttcgcc 4560
ctgcgcgacc cggccggcaa ctgcgtgcac ttcgtggccg aggagcagga
cgtcgagtcc 4620
ggaatggtga gcaagggcga ggaggataac atggccatca tcaaggagtt
catgcgcttc 4680
aaggtgcaca tggagggctc cgtgaacggc cacgagttcg agatcgaggg
cgagggcgag 4740
ggccgcccct acgagggcac ccagaccgcc aagctgaagg tgaccaaggg
tggcccccctg 4800
cccttcgcct gggacatcct gtcccctcag ttcatgtacg gctccaaggc
ctacgtgaag 4860
caccccgccg acatccccga ctacttgaag ctgtccttcc ccgagggctt
caagtgggag 4920
cgcgtgatga acttcgagga cggcggcgtg gtgaccgtga cccaggactc
ctccctgcag 4980
gacggcgagt tcatctacaa ggtgaagctg cgcggcacca acttcccctc
cgacggcccc 5040
gtaatgcaga agaagactat gggctgggag gcctcctccg agcggatgta
ccccgaggac 5100
ggcgccctga agggcgagat caagcagagg ctgaagctga aggacggcgg
ccactacgac 5160
gctgaggtca agaccaccta caaggccaag aagcccgtgc agctgcccgg
cgcctacaac 5220
gtcaacatca agttggacat cacctcccac aacgaggact acaccatcgt
ggaacagtac 5280
gaacgcgccg agggccgcca ctccaccggc ggcatggacg agctgtacaa
gtagactagt 5340
tctagttcta ggggccgaat taattcagat cctcgtgtga gcgttcgcgg
aatcggtcgc 5400
tcgtgtttat gcccgtcttg gtgttgtgct cgcaaggcgg tgcagcagga
taccgtcgcc 5460
ctcctctctc cttgcttctc tgttcttcaa ttcgcgatct cacagaggcc
ggctgtgcac 5520
gcccttcctc accctccttt tcccacctct cggccaccgg tcggctccgt
tccgtctgcc 5580
gtcgagaagg gacgggcatg tgcagctcct ccctttctct cgcgcgcgca
tcttctcttg 5640
cttgtggcac tcacgctcat gcgtcaaggc ggccccacgc cgagcccctg
cgctcccttc 5700
cctcttgcgc atccgtagcg cggatgccgt cgatgcgcaa ggccggcatg
aaggagcgcg 5760
tgccctcaag agggcacact atcatgccct acgtgggcca cgcagcgatg
 
aggccggctt 5820
cggcggagat gcgtcacgca cgtgccagat gatgcgctac gccttccttg
acttgcgccc 5880
ccctctcttc ctccgtctct cactctctct ctctcacaca cacacacaca
cacacacaca 5940
cacaaagctc cggttcgtcc gggccctgca ggcatgccga cgttcggtga
cctgaaccac 6000
ctcgtcgccg ctgtgatgtc tggcgtgacc tgctgcctgc gcttccctgg
ccagctgaac 6060
tctgacctgc gcaagcttct cctgctcaac gacggcgacg gcgctgacga
ggtgcagagt 6120
ggcacagaag ccgcagcggc gtacagcgac gaggagggcg ggaagtcgcc
gagtggtccc 6180
ctggcgaacg actccctgtt catgtccgcg tgggaccgcc gtcgcaacct
cacgagccgc 6240
ccgctgcgca tcacaaccat attcggcccc acctgcgatt cgatggactg
cattctgaag 6300
aagcagccct tcccggagat gaagctcggc gactggcttc tagtgccaga
catgggcagc 6360
tacaccaccg ctgccgcggg agtcttcaac ggtttcgcga cacgccgcct
tgagtttgtg 6420
agctctgttg acttgtctgc gaggtcgagg gctgtgcatg cgcatgaggg
caacactttt 6480
gagttcgtga gcgagtgaaa acggggctga gaggatacaa ggtgctgtag
gatgagatcc 6540
tccgcccacc agagcatggg ctatctcttg tgtctgttct gagcctgcct
ctctctctct 6600
ctcttgcctc cccctccccc ctccccacac acaccccaca cgcacgctct
gcgtcaccct 6660
ctcctccctc agtaacggga gtagacatgc agaagaagta taccactaat
acgtatgcgt 6720
tcctgcaggc atccgagatg gacaccagag caagcgcggg tgtgcgcgag
tggccaggaa 6780
aggggaagag agagaaagag agagagacga aggagcgcgt cccgtcgttg
tgcgtcttct 6840
gtggtggcgc atttaaattt agagcttgac ggggaaagcc ggcgaacgtg
gcgagaaagg 6900
aagggaagaa agcgaaagga gcgggcgcta gggcgctggc aagtgtagcg
gtcacgctgc 6960
gcgtaaccac cacacccgcc gcgcttaatg cgccgctaca gggcgcgtca
ggtggcactt 7020
ttcggggaaa tgtgcgcgga acccctattt gtttattttt ctaaatacat
tcaaatatgt 7080
atccgctcat gagacaataa ccctgataaa tgcttcaata atattgaaaa
aggaagagta 7140
tgagtattca acatttccgt gtcgccctta ttcccttttt tgcggcattt
tgccttcctg 7200
tttttgctca cccagaaacg ctggtgaaag taaaagatgc tgaagatcag
ttgggtgcac 7260
gagtgggtta catcgaactg gatctcaaca gcggtaagat ccttgagagt
tttcgccccg 7320
aagaacgttt tccaatgatg agcactttta aagttctgct atgtggcgcg
gtattatccc 7380
gtattgacgc cgggcaagag caactcggtc gccgcataca ctattctcag
aatgacttgg 7440
ttgagtactc accagtcaca gaaaagcatc ttacggatgg catgacagta
agagaattat 7500
gcagtgctgc cataaccatg agtgataaca ctgcggccaa cttacttctg
acaacgatcg 7560
gaggaccgaa ggagctaacc gcttttttgc acaacatggg ggatcatgta
actcgccttg 7620
atcgttggga accggagctg aatgaagcca taccaaacga cgagcgtgac
accacgatgc 7680
 
ctgtagcaat ggcaacaacg ttgcgcaaac tattaactgg cgaactactt
actctagctt 7740
cccggcaaca attaatagac tggatggagg cggataaagt tgcaggacca
cttctgcgct 7800
cggcccttcc ggctggctgg tttattgctg ataaatctgg agccggtgag
cgtgggtctc 7860
gcggtatcat tgcagcactg gggccagatg gtaagccctc ccgtatcgta
gttatctaca 7920
cgacggggag tcaggcaact atggatgaac gaaatagaca gatcgctgag
ataggtgcct 7980
cactgattaa gcattggtaa ctgtcagacc aagtttactc atatatactt
tagattgatt 8040
taaaacttca tttttaattt aaaaggatct aggtgaagat cctttttgat
aatctcatga 8100
ccaaaatccc ttaacgtgag ttttcgttcc actgagcgtc agaccccgta
gaaaagatca 8160
aaggatcttc ttgagatcct ttttttctgc gcataatctg ctgcttgcaa
acaaaaaaac 8220
caccgctacc agcggtggtt tgtttgccgg atcaagagct accaactctt
tttccaaggt 8280
aactggcttc agcagagcgc agataccaaa tactgttctt ctagtgtagc
cgtagttagg 8340
ccaccacttc aagaactctg tagcaccgcc tacatacctc gctctgctaa
tcctgttacc 8400
agtggctgct gccagtggcg ataagtcgtg tcttaccggg ttggactcaa
gacgatagtt 8460
accggataag gcgcagcggt cgggctgaac ggggggttcg tgcacacagc
ccagcttgga 8520
gcgaacgacc tacaccgaac tgagatacct acagcgtgag ctatgagaaa
gcgccacgct 8580
tcccgaaggg agaaaggcgg acaggtatcc ggtaagcggc agggtcggaa
caggagagcg 8640
cacgagggag cttccagggg gaaacgcctg gtatctttat agtcctgtcg
ggtttcgcca 8700
cctctgactt gagcgtcgat ttttgtgatg ctcgtcaggg gggcggagcc
tatggaaaaa 8760
cgccagcaac gcggcctttt tacggttcct ggccttttgc tggcctt
8807
<210> 10
   <211> DNA
   <212> 8957
   <213> Leishmania tarentolae
<400> 1 
atttaaatga atacttacaa ggtactcctg cacccggcac aagcgtatgt
aattgctagc 60
acgccggtgc cgacccaacc tccaactccc gtgctcacgc gcgcacacac
gcacgcacaa 120
gcatcatcat catcatcatc atggtcatcc ctcccttatc tggcgaagga
ggcactgtga 180
cgcaaggttt tctgtgtctc tcctacccct ctactgattc cttctttctc
tcttcctctt 240
ctcgcactgc acccccctcc ccccccccac acacacacac ttgctgtcat
ggctgtgcgc 300
attcgctcac tcttgtcgtg tcccgtttcc ctcatggcgt cgtcgtcttc
ccctgcgcaa 360
ccacccacgc tgcacacgca cgctccacag cgcgcttcgc cttcttcgct
ctctccctag 420
cctcctctct gtcaccctcc actgccgccg cggttggccg ctctgcatcc
ttttcccgca 480
 
gcctttcctt    ttttttttca    ctgtcagtct    ggctcatacc    cgtggagctg
tgtctccccg    540
agcttcccat    acacccaccc    cccacgacct    ttctcagctt    cctttctcag
tcgcttgagc    600
acgcagtgca    ttcacttctt    cgtccctcag    ttcgcctcgc    tctcgctctc
tctcgcgcgc    660
gctgccgtac    ccgcccctcc    tcgtactccc    ctccttcctc    atccccaccc
cccccgtgtg    720
cgccggctcc    tctctgaatt    ctaatacgac    tcactatagg    gagatcgtcc
ctatcagtga    780
tagagatcca    gtcgcagcct    gaccgcatca    cacatcaagg    cgttacagcc
tctccgtctt    840
tgctgcaccc    aagtgtattc    gtgtgaccgc    gttgagggat    ctaatggatt
cggaactttg    900
gtcgtttggt    ctctcatgct    gcccaatccc    atgcgctttt    ctccgtgcct
ctctgtctcc    960
ccttccgttc    tatttggaca    acgatgtgta    ctgtaatgcg    tgcgccatct
gacaacgaat    1020
agatcagcag    cattcgcaca    cttgcacata    cccagtgaag    cttttgtgtc
tgtcgtattg    1080
acaacaccga    ctgcaacaag    gtgtagatag    aagttggcct    tctcgctcgc
tcgcacgctc    1140
ttcacgctcc    tgctttcctt    gctgtgcctt    gccaccagat    ctgccatggc
catgcagagc    1200
tggggtacaa    tcatatgcat    tcgaattctc    ttgcgatttc    ttctgctctg
ggtgcttatc    1260
gggaactcac    acactgaaga    agacatcata    attacaacca    agaacggaaa
agtcagaggg    1320
atgaacttgc    cagttcttgg    tggcacagta    acagcctttc    ttgggattcc
ctatgcacag    1380
ccgcctcttg    gtagacttcg    attcaaaaag    ccacaatcct    tgactaagtg
gtccaatatt    1440
tggaatgcca    caaaatatgc    caattcttgc    tatcagaaca    cagatcaaag
tttcccaggc    1500
ttccttggat    cagagatgtg    gaacccaaac    actgaactta    gtgaagactg
tttatatctg    1560
aatgtgtgga    ttccagcacc    taaaccaaaa    aatgctactg    taatgatatg
gatctatggt    1620
ggtggttttc    aaactgggac    atcatctttg    cctgtttatg    atggcaagtt
tctggctcgg    1680
gttgaaagag    ttattgtagt    ttcaatgaac    tatagagtgg    gtgccctagg
attcttagcc    1740
ttatcagaaa    atcctgaggc    accagggaac    atgggcttat    ttgatcaaca
gttggcactt    1800
cagtgggtcc    aaaaaaatat    agcagccttt    ggtggtaatc    ctagaagtgt
aactctcttt    1860
ggagaaagtg    caggagcagc    ttcagttagc    cttcatttac    tttctcctag
aagccagcct    1920
ttgtttacca    gagccattct    gcaaagtgga    tcctctaatg    ccccttgggc
agtaacatct    1980
ctgtatgaag    ctaggaacag    aacattgacc    ctagctaaac    gtatgggttg
ctctagggac    2040
aatgagactg    agatgatcaa    atgtcttcga    gacaaagatc    cccaggaaat
tcttctgaat    2100
gaagtatttg    ttgtccccta    tgatactctc    ttgtcagtaa    actttggtcc
aactgtggat    2160
ggcgattttc    tcactgacat    gccagataca    ctactccaac    ttggacagtt
caaaagaacc    2220
cagatcttgg    tgggtgttaa    taaagatgaa    gggacagcat    ttttagtata
tggggctcct    2280
ggtttcagca    aagataacaa    cagtatcata    acaagaaaag    aatttcagga
gggtttaaaa    2340
atattttttc    caagagtgag    tgagtttgga    agagaatcaa    tcctttttca
 
ttacatggac 2400
tggttagatg atcagagagc cgaaaactac agagaggcct tggatgatgt
tgttggggat 2460
tacaatatca tatgccctgc cttggagttc accaaaaagt tctcagaatt
gggaaatgat 2520
gcctttttct actattttga acaccgatcg accaaacttc cttggccaga
atggatggga 2580
gtgatgcatg gttacgaaat cgaatttgtc tttggtttac ctctggaaag
aagagttaat 2640
tacacaaaag ctgaggaaat tttgagtaga tccattatga aacgctgggc
aaatttgca 2700
aaatatggaa atccaaatgg gacccagagc aatagcacaa gatggcctgt
cttcaagagc 2760
actgaacaaa aatatttaac cttgaataca gagtcaccaa aagtgtacac
caaactacga 2820
gctcaacaat gtcgattctg gacactattt tttcctaaag tcttggaatt
gacaggaaat 2880
attgatgaag cagaacgaga atggaaagca ggattccatc gctggaacaa
ttacatgatg 2940
gactggaaaa atcaatttaa cgattacact agcaagaaag aaagctgttc
agatttctt 3000
aagggtaccc accaccatca ccaccactag gcggccgccc tcctcctcct
ttcttgttcc 3060
tttcacgtcg ccttctcggt tgtagctggc agacgacgag tcttactttt
acgtgtactt 3120
ctctatagat gatgtatgat ctctctgcat gcgtgttcgt gcatgtgtcc
gtgtgttgtg 3180
tacgcgtgcg tctcgcctca gctctccgcg tgaaagggtt tgactgccca
tgatgcgtgt 3240
gtatatccac gcgcaggcac gcacacacac acacacacac acacacacag
gcacacacag 3300
gcacacaaac gcatctcagg ccgagccgca tacgtctctc gcacggtctc
gtttatttga 3360
tcatgtagtt gagtattaaa ttgggaagac aaaaacataa tagcacgaag
agtcgggcac 3420
gaaaagcccg atctctctct ctctctctcg tgcgcgcagg ggcgtgtggg
tgcacgacga 3480
cgaggacgga gggggaaggg gaggcataaa cggatcgatc ccccatcgca
catgcgggta 3540
cgagcattat ctgctgtgtc tggtccttta tcatatcgct acccgcccgc
cccccgcccc 3600
cctccccccc cccccccccg cggctcctgt cactgtcgtt cctgcgactc
cccacacacc 3660
cgcgcaacgc tcatgtcacg aagagaaaag tagaaggcgt ggcgatgcgt
tgcgcggccc 3720
cctgtccgct ggcatgcaga cgtcgagccg tggaacggat ggtagcgaga
gacaacgcgg 3780
cgatgcagga aaggagattt actgcaggac gtctacacac gcgcgcacac
cactgcgaat 3840
ggcaccgtgc tgaggaaaat tgggggggag ggcgcacggg ggcggggcag
gaaacggttg 3900
gatcagcagc actctcaact tgtgtccgta ttgcgaagga gggagaggag
cctgcccgta 3960
ttatgggcgc tgaaacgtcg gaaccaccta tgaatcccac ttctgcgtcg
cggaggcgta 4020
ctgcttccga ccacctccgc agctcacgcg cgtcgagctc cctccctttc
cccttctttc 4080
cgttgtgtgt gtgtgtgtgt gtgtgtcttg tcacgatgca tggccatctc
tcctccaacc 4140
ctccacgcct tcctctcccc cgcccatcaa acgcgctacg gccacaacca
tttcttcaag 4200
tatcacatcc accaccactc ttacccacct accctcttcg actctgacac
agccactgac 4260
 
gccctctccg cctctctcgc tcgctgcaca ttgactctcc acagcccccc
ctccctcgca 4320
cagccagccc cacccaccca cccacccacc caccgctcta cacacctcca
cagcagccgg 4380
atccaccgca tggccaagtt gaccagtgcc gttccggtgc tcaccgcgcg
cgacgtcgcc 4440
ggagcggtcg agttctggac cgaccggctc gggttctccc gggacttcgt
ggaggacgac 4500
ttcgccggtg tggtccggga caacgtgacc ctgttcatca gcgcggtcca
ggaccaggtg 4560
gtgccggaca acaccctggc ctgggtgtgg gtgcgcggcc tggacgagct
gtacgccgag 4620
tggtcggagg tcgtgtccac gaacttccgg gacgcctccg ggccggccat
gaccgagatc 4680
ggcgagcagc cgtgggggcg ggagttcgcc ctgcgcgacc cggccggcaa
ctgcgtgcac 4740
ttcgtggccg aggagcagga cgtcgagtcc ggaatggtga gcaagggcga
ggaggataac 4800
atggccatca tcaaggagtt catgcgcttc aaggtgcaca tggagggctc
cgtgaacggc 4860
cacgagttcg agatcgaggg cgagggcgag ggccgcccct acgagggcac
ccagaccgcc 4920
aagctgaagg tgaccaaggg tggccccctg cccttcgcct gggacatcct
gtcccctcag 4980
ttcatgtacg gctccaaggc ctacgtgaag caccccgccg acatccccga
ctacttgaag 5040
ctgtccttcc ccgagggctt caagtgggag cgcgtgatga acttcgagga
cggcggcgtg 5100
gtgaccgtga cccaggactc ctccctgcag gacggcgagt tcatctacaa
ggtgaagctg 5160
cgcggcacca acttcccctc cgacggcccc gtaatgcaga agaagactat
gggctgggag 5220
gcctcctccg agcggatgta ccccgaggac ggcgccctga agggcgagat
caagcagagg 5280
ctgaagctga aggacggcgg ccactacgac gctgaggtca agaccaccta
caaggccaag 5340
aagcccgtgc agctgcccgg cgcctacaac gtcaacatca agttggacat
cacctcccac 5400
aacgaggact acaccatcgt ggaacagtac gaacgcgccg agggccgcca
ctccaccggc 5460
ggcatggacg agctgtacaa gtagactagt tctagttcta ggggccgaat
taattcagat 5520
cctcgtgtga gcgttcgcgg aatcggtcgc tcgtgtttat gcccgtcttg
gtgttgtgct 5580
cgcaaggcgg tgcagcagga taccgtcgcc ctcctctctc cttgcttctc
tgttcttcaa 5640
ttcgcgatct cacagaggcc ggctgtgcac gcccttcctc accctccttt
tcccacctct 5700
cggccaccgg tcggctccgt tccgtctgcc gtcgagaagg gacgggcatg
tgcagctcct 5760
ccctttctct cgcgcgcgca tcttctcttg cttgtggcac tcacgctcat
gcgtcaaggc 5820
ggccccacgc cgagcccctg cgctcccttc cctcttgcgc atccgtagcg
cggatgccgt 5880
cgatgcgcaa ggccggcatg aaggagcgcg tgccctcaag agggcacact
atcatgccct 5940
acgtgggcca cgcagcgatg aggccggctt cggcggagat gcgtcacgca
cgtgccagat 6000
gatgcgctac gccttccttg acttgcgccc ccctctcttc ctccgtctct
cactctctct 6060
ctctcacaca cacacacaca cacacacaca cacaaagctc cggttcgtcc
gggccctgca 6120
ggcatgccga cgttcggtga cctgaaccac ctcgtcgccg ctgtgatgtc
 
tggcgtgacc 6180
tgctgcctgc gcttccctgg ccagctgaac tctgacctgc gcaagcttct
cctgctcaac 6240
gacggcgacg gcgctgacga ggtgcagagt ggcacagaag ccgcagcggc
gtacagcgac 6300
gaggagggcg ggaagtcgcc gagtggtccc ctggcgaacg actccctgtt
catgtccgcg 6360
tgggaccgcc gtcgcaacct cacgagccgc ccgctgcgca tcacaaccat
attcggcccc 6420
acctgcgatt cgatggactg cattctgaag aagcagccct tcccggagat
gaagctcggc 6480
gactggcttc tagtgccaga catgggcagc tacaccaccg ctgccgcggg
agtcttcaac 6540
ggtttcgcga cacgccgcct tgagtttgtg agctctgttg acttgtctgc
gaggtcgagg 6600
gctgtgcatg cgcatgaggg caacactttt gagttcgtga gcgagtgaaa
acggggctga 6660
gaggatacaa ggtgctgtag gatgagatcc tccgcccacc agagcatggg
ctatctcttg 6720
tgtctgttct gagcctgcct ctctctctct ctcttgcctc cccctccccc
ctccccacac 6780
acaccccaca cgcacgctct gcgtcaccct ctcctccctc agtaacggga
gtagacatgc 6840
agaagaagta taccactaat acgtatgcgt tcctgcaggc atccgagatg
gacaccagag 6900
caagcgcggg tgtgcgcgag tggccaggaa aggggaagag agagaaagag
agagagacga 6960
aggagcgcgt cccgtcgttg tgcgtcttct gtggtggcgc atttaaattt
agagcttgac 7020
ggggaaagcc ggcgaacgtg gcgagaaagg aagggaagaa agcgaaagga
gcgggcgcta 7080
gggcgctggc aagtgtagcg gtcacgctgc gcgtaaccac cacacccgcc
gcgcttaatg 7140
cgccgctaca gggcgcgtca ggtggcactt ttcggggaaa tgtgcgcgga
acccctattt 7200
gtttattttt ctaaatacat tcaaatatgt atccgctcat gagacaataa
ccctgataaa 7260
tgcttcaata atattgaaaa aggaagagta tgagtattca acatttccgt
gtcgccctta 7320
ttcccttttt tgcggcattt tgccttcctg tttttgctca cccagaaacg
ctggtgaaag 7380
taaaagatgc tgaagatcag ttgggtgcac gagtgggtta catcgaactg
gatctcaaca 7440
gcggtaagat ccttgagagt tttcgccccg aagaacgttt tccaatgatg
agcactttta 7500
aagttctgct atgtggcgcg gtattatccc gtattgacgc cgggcaagag
caactcggtc 7560
gccgcataca ctattctcag aatgacttgg ttgagtactc accagtcaca
gaaaagcatc 7620
ttacggatgg catgacagta agagaattat gcagtgctgc cataaccatg
agtgataaca 7680
ctgcggccaa cttacttctg acaacgatcg gaggaccgaa ggagctaacc
gcttttttgc 7740
acaacatggg ggatcatgta actcgccttg atcgttggga accggagctg
aatgaagcca 7800
taccaaacga cgagcgtgac accacgatgc ctgtagcaat ggcaacaacg
ttgcgcaaac 7860
tattaactgg cgaactactt actctagctt cccggcaaca attaatagac
tggatggagg 7920
cggataaagt tgcaggacca cttctgcgct cggcccttcc ggctggctgg
tttattgctg 7980
ataaatctgg agccggtgag cgtgggtctc gcggtatcat tgcagcactg
gggccagatg 8040
 
gtaagccctc ccgtatcgta gttatctaca cgacggggag tcaggcaact
atggatgaac 8100
gaaatagaca gatcgctgag ataggtgcct cactgattaa gcattggtaa
ctgtcagacc 8160
aagtttactc atatatactt tagattgatt taaaacttca tttttaattt
aaaaggatct 8220
aggtgaagat cctttttgat aatctcatga ccaaaatccc ttaacgtgag
ttttcgttcc 8280
actgagcgtc agaccccgta gaaaagatca aaggatcttc ttgagatcct
ttttttctgc 8340
gcataatctg ctgcttgcaa acaaaaaaac caccgctacc agcggtggtt
tgtttgccgg 8400
atcaagagct accaactctt tttccaaggt aactggcttc agcagagcgc
agataccaaa 8460
tactgttctt ctagtgtagc cgtagttagg ccaccacttc aagaactctg
tagcaccgcc 8520
tacatacctc gctctgctaa tcctgttacc agtggctgct gccagtggcg
ataagtcgtg 8580
tcttaccggg ttggactcaa gacgatagtt accggataag gcgcagcggt
cgggctgaac 8640
ggggggttcg tgcacacagc ccagcttgga gcgaacgacc tacaccgaac
tgagatacct 8700
acagcgtgag ctatgagaaa gcgccacgct tcccgaaggg agaaaggcgg
acaggtatcc 8760
ggtaagcggc agggtcggaa caggagagcg cacgagggag cttccagggg
gaaacgcctg 8820
gtatctttat agtcctgtcg ggtttcgcca cctctgactt gagcgtcgat
ttttgtgatg 8880
ctcgtcaggg gggcggagcc tatggaaaaa cgccagcaac gcggcctttt
tacggttcct 8940
ggccttttgc tggcctt
8957
<210> 11
   <211> DNA
   <212> 9137
   <213> Leishmania tarentolae
<400> 1

atttaaatga atacttacaa ggtactcctg cacccggcac aagcgtatgt
aattgctagc 60
acgccggtgc cgacccaacc tccaactccc gtgctcacgc gcgcacacac
gcacgcacaa 120
gcatcatcat catcatcatc atggtcatcc ctcccttatc tggcgaagga
ggcactgtga 180
cgcaaggttt tctgtgtctc tcctacccct ctactgattc cttctttctc
tcttcctctt 240
ctcgcactgc acccccctcc ccccccccac acacacacac ttgctgtcat
ggctgtgcgc 300
attcgctcac tcttgtcgtg tcccgtttcc ctcatggcgt cgtcgtcttc
ccctgcgcaa 360
ccacccacgc tgcacacgca cgctccacag cgcgcttcgc cttcttcgct
ctctccctag 420
cctcctctct gtcaccctcc actgccgccg cggttggccg ctctgcatcc
ttttcccgca 480
gcctttcctt ttttttttca ctgtcagtct ggctcatacc cgtggagctg
tgtctccccg 540
agcttcccat acacccaccc cccacgacct ttctcagctt cctttctcag
tcgcttgagc 600
acgcagtgca ttcacttctt cgtccctcag ttcgcctcgc tctcgctctc
tctcgcgcgc 660

gctgccgtac ccgcccctcc tcgtactccc ctccttcctc atccccaccc
cccccgtgtg 720
cgccggctcc tctctgaatt ctaatacgac tcactatagg gagatcgtcc
ctatcagtga 780
tagagatcca gtcgcagcct gaccgcatca cacatcaagg cgttacagcc
tctccgtctt 840
tgctgcaccc aagtgtattc gtgtgaccgc gttgagggat ctaatggatt
cggaactttg 900
gtcgtttggt ctctcatgct gcccaatccc atgcgctttt ctccgtgcct
ctctgtctcc 960
ccttccgttc tatttggaca acgatgtgta ctgtaatgcg tgcgccatct
gacaacgaat 1020
agatcagcag cattcgcaca cttgcacata cccagtgaag cttttgtgtc
tgtcgtattg 1080
acaacaccga ctgcaacaag gtgtagatag aagttggcct tctcgctcgc
tcgcacgctc 1140
ttcacgctcc tgctttcctt gctgtgcctt gccaccagat ctgccatggc
catgcagtcc 1200
tggggtacca tcatctgcat ccgtatcctg ctgcgtttcc tgctgctgtg
ggttctgatc 1260
ggtaactccc acaccgaaga agacatcatc atcaccacca aaaacggtaa
agttcgtggt 1320
atgaacctgc cggttctggg tggtaccgtt accgctttcc tgggtatccc
gtacgctcag 1380
ccgccgctgg gtcgtctgcg tttcaaaaaa ccgcagtccc tgaccaaatg
gtccaacatc 1440
tggaacgcta ccaaatacgc taactcctgc taccagaaca ccgaccagtc
cttcccgggt 1500
ttcctgggtt ccgaaatgtg gaacccgaac accgaactgt ccgaagactg
cctgtacctg 1560
aacgtttgga tcccggctcc gaaaccgaaa aacgctaccg ttatgatctg
gatctacggt 1620
ggtggtttcc agaccggtac ctcctccctg ccggtttacg acggtaaatt
cctggctcgt 1680
gttgaacgtg ttatcgttgt ttccatgaac taccgtgttg gtgctctggg
tttcctggct 1740
ctgtccgaaa acccggaagc tccgggtaac atgggtctgt tcgaccagca
gctggctctg 1800
cagtgggttc agaaaaacat cgctgctttc ggtggtaacc cgcgttccgt
taccctgttc 1860
ggtgaatccg ctggtgctgc ttccgtttcc ctgcacctgc tgtccccgcg
ttcccagccg 1920
ctgttcaccc gtgctatcct gcagtccggt tcctccaacg ctccgtgggc
tgttacctcc 1980
ctgtacgaag ctcgtaaccg taccctgacc ctggctaaac gtatgggttg
ctcccgtgac 2040
aacgaaaccg aaatgatcaa atgcctgcgt gacaaagacc cgcaggaaat
cctgctgaac 2100
gaagttttcg ttgttccgta cgacaccctg ctgtccgtta acttcggtcc
gaccgttgac 2160
ggtgacttcc tgaccgacat gccggacacc ctgctgcagc tgggtcagtt
caaacgtacc 2220
cagatcctgg ttggtgttaa caaagacgaa ggtaccgctt tcctggttta
cggtgctccg 2280
ggtttctcca aagacaacaa ctccatcatc acccgtaaag aattccagga
aggtctgaaa 2340
atcttcttcc cgcgtgtttc cgaattcggt cgtgaatcca tcctgttcca
ctacatggac 2400
tggctggacg accagcgtgc tgaaaactac cgtgaagctc tggacgacgt
tgttggtgac 2460
tacaacatca tctgcccggc tctggaattc accaaaaaat tctccgaact
gggtaacgac 2520
gctttcttct actacttcga acaccgttcc accaaactgc cgtggccgga

atggatgggt 2580
gttatgcacg gttacgaaat cgaattcgtt ttcggtctgc cgctggaacg
tcgtgttaac 2640
tacaccaaag ctgaagaaat cctgtcccgt tccatcatga aacgttgggc
taacttcgct 2700
aaatacggta acccgaacgg tacccagtcc aactccaccc gttggccggt
tttcaaatcc 2760
accgaacaga aatacctgac cctgaacacc gaatccccga aagtttacac
caaactgcgt 2820
gctcagcagt gccgtttctg gaccctgttc ttcccgaaag ttctggaact
gaccggtaac 2880
atcgacgaag ctgaacgtga atggaaagct ggtttccacc gttggaacaa
ctacatgatg 2940
gactggaaaa accagttcaa cgactacacc tccaaaaaag aatcctgctc
cgacttcctt 3000
aagggtaccc accaccatca ccaccactag gcggccgccc tcctcctcct
ttcttgttcc 3060
tttcacgtcg ccttctcggt tgtagctggc agacgacgag tcttactttt
acgtgtactt 3120
ctctatagat gatgtatgat ctctctgcat gcgtgttcgt gcatgtgtcc
gtgtgttgtg 3180
tacgcgtgcg tctcgcctca gctctccgcg tgaaagggtt tgactgccca
tgatgcgtgt 3240
gtatatccac gcgcaggcac gcacacacac acacacacac acacacacag
gcacacacag 3300
gcacacaaac gcatctcagg ccgagccgca tacgtctctc gcacggtctc
gtttatttga 3360
tcatgtagtt gagtattaaa ttgggaagac aaaaacataa tagcacgaag
agtcgggcac 3420
gaaaagcccg atctctctct ctctctctcg tgcgcgcagg ggcgtgtggg
tgcacgacga 3480
cgaggacgga gggggaaggg gaggcataaa cggatcgatc ccccatcgca
catgcgggta 3540
cgagcattat ctgctgtgtc tggtccttta tcatatcgct acccgcccgc
cccccgcccc 3660
cctccccccc cccccccccg cggctcctgt cactgtcgtt cctgcgactc
cccacacacc 3720
cgcgcaacgc tcatgtcacg aagagaaaag tagaaggcgt ggcgatgcgt
tgcgcggccc 3780
cctgtccgct ggcatgcaga cgtcgagccg tggaacggat ggtagcgaga
gacaacgcgg 3840
cgatgcagga aaggagattt actgcaggac gtctacacac gcgcgcacac
cactgcgaat 3900
ggcaccgtgc tgaggaaaat tgggggggag ggcgcacggg ggcggggcag
gaaacggttg 3960
gatcagcagc actctcaact tgtgtccgta ttgcgaagga gggagaggag
cctgcccgta 4020
ttatgggcgc tgaaacgtcg gaaccaccta tgaatcccac ttctgcgtcg
cggaggcgta 4080
ctgcttccga ccacctccgc agctcacgcg cgtcgagctc cctccctttc
cccttctttc 4140
cgttgtgtgt gtgtgtgtgt gtgtgtcttg tcacgatgca tggccatctc
tcctccaacc 4200
ctccacgcct tcctctcccc cgcccatcaa acgcgctacg gccacaacca
tttcttcaag 4260
tatcacatcc accaccactc ttacccacct accctcttcg actctgacac
agccactgac 4320
gccctctccg cctctctcgc tcgctgcaca ttgactctcc acagcccccc
ctccctcgca 4440
cagccagccc cacccaccca cccacccacc caccgctcta cacacctcca
cagcagccgg 4500
atccaccgca tggccaagtt gaccagtgcc gttccggtgc tcaccgcgcg
cgacgtcgcc 4560

ggagcggtcg agttctggac cgaccggctc gggttctccc gggacttcgt
ggaggacgac 4620
ttcgccggtg tggtccggga caacgtgacc ctgttcatca gcgcggtcca
ggaccaggtg 4680
gtgccggaca acaccctggc ctgggtgtgg gtgcgcggcc tggacgagct
gtacgccgag 4740
tggtcggagg tcgtgtccac gaacttccgg gacgcctccg ggccggccat
gaccgagatc 4800
ggcgagcagc cgtgggggcg ggagttcgcc ctgcgcgacc cggccggcaa
ctgcgtgcac 4860
ttcgtggccg aggagcagga cgtcgagtcc ggaatggtga gcaagggcga
ggaggataac 4920
atggccatca tcaaggagtt catgcgcttc aaggtgcaca tggagggctc
cgtgaacggc 5040
cacgagttcg agatcgaggg cgagggcgag ggccgcccct acgagggcac
ccagaccgcc 5100
aagctgaagg tgaccaaggg tggccccctg cccttcgcct gggacatcct
gtcccctcag 5160
ttcatgtacg gctccaaggc ctacgtgaag caccccgccg acatccccga
ctacttgaag 5220
ctgtccttcc ccgagggctt caagtgggag cgcgtgatga acttcgagga
cggcggcgtg 5280
gtgaccgtga cccaggactc ctccctgcag gacggcgagt tcatctacaa
ggtgaagctg 5340
cgcggcacca acttcccctc cgacggcccc gtaatgcaga agaagactat
gggctgggag 5400
gcctcctccg agcggatgta ccccgaggac ggcgccctga agggcgagat
caagcagagg 5460
ctgaagctga aggacggcgg ccactacgac gctgaggtca agaccaccta
caaggccaag 5520
aagcccgtgc agctgcccgg cgcctacaac gtcaacatca agttggacat
cacctcccac 5580
aacgaggact acaccatcgt ggaacagtac gaacgcgccg agggccgcca
ctccaccggc 5640
ggcatggacg agctgtacaa gtagactagt tctagttcta ggggccgaat
taattcagat 5700
cctcgtgtga gcgttcgcgg aatcggtcgc tcgtgtttat gcccgtcttg
gtgttgtgct 5760
cgcaaggcgg tgcagcagga taccgtcgcc ctcctctctc cttgcttctc
tgttcttcaa 5820
ttcgcgatct cacagaggcc ggctgtgcac gcccttcctc accctccttt
tcccacctct 5880
cggccaccgg tcggctccgt tccgtctgcc gtcgagaagg gacgggcatg
tgcagctcct 5940
ccctttctct cgcgcgcgca tcttctcttg cttgtggcac tcacgctcat
gcgtcaaggc 6000
ggccccacgc cgagcccctg cgctcccttc cctcttgcgc atccgtagcg
cggatgccgt 6060
cgatgcgcaa ggccggcatg aaggagcgcg tgccctcaag agggcacact
atcatgccct 6120
acgtgggcca cgcagcgatg aggccggctt cggcggagat gcgtcacgca
cgtgccagat 6180
gatgcgctac gccttccttg acttgcgccc ccctctcttc ctccgtctct
cactctctct 6240
ctctcacaca cacacacaca cacacacaca cacaaagctc cggttcgtcc
gggccctgca 6300
ggcatgccga cgttcggtga cctgaaccac ctcgtcgccg ctgtgatgtc
tggcgtgacc 6360
tgctgcctgc gcttccctgg ccagctgaac tctgacctgc gcaagcttct
cctgctcaac 6420
gacggcgacg gcgctgacga ggtgcagagt ggcacagaag ccgcagcggc
gtacagcgac 6480
gaggagggcg ggaagtcgcc gagtggtccc ctggcgaacg actccctgtt

catgtccgcg 6540
tgggaccgcc gtcgcaacct cacgagccgc ccgctgcgca tcacaaccat
attcggcccc 6600
acctgcgatt cgatggactg cattctgaag aagcagccct tcccggagat
gaagctcggc 6660
gactggcttc tagtgccaga catgggcagc tacaccaccg ctgccgcggg
agtcttcaac 6720
ggtttcgcga cacgccgcct tgagtttgtg agctctgttg acttgtctgc
gaggtcgagg 6780
gctgtgcatg cgcatgaggg caacactttt gagttcgtga gcgagtgaaa
acggggctga 6840
gaggatacaa ggtgctgtag gatgagatcc tccgcccacc agagcatggg
ctatctcttg 6900
tgtctgttct gagcctgcct ctctctctct ctcttgcctc cccctccccc
ctccccacac 6960
acaccccaca cgcacgctct gcgtcaccct ctcctccctc agtaacggga
gtagacatgc 7020
agaagaagta taccactaat acgtatgcgt tcctgcaggc atccgagatg
gacaccagag 7080
caagcgcggg tgtgcgcgag tggccaggaa aggggaagag agagaaagag
agagagacga 7140
aggagcgcgt cccgtcgttg tgcgtcttct gtggtggcgc atttaaattt
agagcttgac 7200
ggggaaagcc ggcgaacgtg gcgagaaagg aagggaagaa agcgaaagga
gcgggcgcta 7260
gggcgctggc aagtgtagcg gtcacgctgc gcgtaaccac cacacccgcc
gcgcttaatg 7320
cgccgctaca gggcgcgtca ggtggcactt ttcggggaaa tgtgcgcgga
acccctattt 7380
gtttattttt ctaaatacat tcaaatatgt atccgctcat gagacaataa
ccctgataaa 7440
tgcttcaata atattgaaaa aggaagagta tgagtattca acatttccgt
gtcgccctta 7500
ttcccttttt tgcggcattt tgccttcctg tttttgctca cccagaaacg
ctggtgaaag 7560
taaaagatgc tgaagatcag ttgggtgcac gagtgggtta catcgaactg
gatctcaaca 7620
gcggtaagat ccttgagagt tttcgccccg aagaacgttt tccaatgatg
agcactttta 7680
aagttctgct atgtggcgcg gtattatccc gtattgacgc cgggcaagag
caactcggtc 7740
gccgcataca ctattctcag aatgacttgg ttgagtactc accagtcaca
gaaaagcatc 7800
ttacggatgg catgacagta agagaattat gcagtgctgc cataaccatg
agtgataaca 7860
ctgcggccaa cttacttctg acaacgatcg gaggaccgaa ggagctaacc
gcttttttgc 7920
acaacatggg ggatcatgta actcgccttg atcgttggga accggagctg
aatgaagcca 7980
taccaaacga cgagcgtgac accacgatgc ctgtagcaat ggcaacaacg
ttgcgcaaac 8040
tattaactgg cgaactactt actctagctt cccggcaaca attaatagac
tggatggagg 8100
cggataaagt tgcaggacca cttctgcgct cggcccttcc ggctggctgg
tttattgctg 8160
ataaatctgg agccggtgag cgtgggtctc gcggtatcat tgcagcactg
gggccagatg 8220
gtaagccctc ccgtatcgta gttatctaca cgacggggag tcaggcaact
atggatgaac 8280
gaaatagaca gatcgctgag ataggtgcct cactgattaa gcattggtaa
ctgtcagacc 8340
aagtttactc atatatactt tagattgatt taaaacttca tttttaattt
aaaaggatct 8400

aggtgaagat cctttttgat aatctcatga ccaaaatccc ttaacgtgag
ttttcgttcc 8460
actgagcgtc agaccccgta gaaaagatca aaggatcttc ttgagatcct
ttttttctgc 8520
gcataatctg ctgcttgcaa acaaaaaaac caccgctacc agcggtggtt
tgtttgccgg 8580
atcaagagct accaactctt tttccaaggt aactggcttc agcagagcgc
agataccaaa 8640
tactgttctt ctagtgtagc cgtagttagg ccaccacttc aagaactctg
tagcaccgcc 8700
tacatacctc gctctgctaa tcctgttacc agtggctgct gccagtggcg
ataagtcgtg 8760
tcttaccggg ttggactcaa gacgatagtt accggataag gcgcagcggt
cgggctgaac 8820
ggggggttcg tgcacacagc ccagcttgga gcgaacgacc tacaccgaac
tgagatacct 8880
acagcgtgag ctatgagaaa gcgccacgct tcccgaaggg agaaaggcgg
acaggtatcc 8940
ggtaagcggc agggtcggaa caggagagcg cacgagggag cttccagggg
gaaacgcctg 9000
gtatctttat agtcctgtcg ggtttcgcca cctctgactt gagcgtcgat
ttttgtgatg 9060
ctcgtcaggg gggcggagcc tatggaaaaa cgccagcaac gcggcctttt
tacggttcct 9120
ggccttttgc tggcctt
9137